(19) Europäisches Patentamt
European Patent Office
Office européen des brevets

(11) Veröffentlichungsnummer: **0 245 209**
**A1**

(12) # EUROPÄISCHE PATENTANMELDUNG

(21) Anmeldenummer: **87810282.1**

(22) Anmeldetag: **01.05.87**

(51) Int. Cl.[4]: **C 07 D 493/22**, C 07 F 7/04, A 61 K 31/35, A 01 N 43/92 // (C07D493/22, 313:00, 311:00, 311:00, 307:00)

(30) Priorität: **07.05.86 CH 1864/86**

(43) Veröffentlichungstag der Anmeldung: **11.11.87 Patentblatt 87/46**

(84) Benannte Vertragsstaaten: **AT BE CH DE ES FR GB GR IT LI LU NL SE**

(71) Anmelder: **CIBA-GEIGY AG, Klybeckstrasse 141, CH-4002 Basel (CH)**

(72) Erfinder: **Maienfisch, Peter, Dr., Traugott Meyer-Strasse 5, CH-4147 Aesch (CH)**

(54) **Milbemycin-Derivate, ihre Herstellung und ihre Verwendung zur Bekämpfung von Schädlingen.**

(57) Neue Verbindungen der Formel

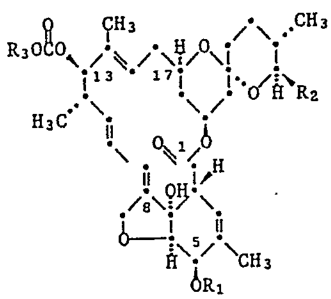

(I)

in welcher
$R_1$ Wasserstoff, eine Silyl- oder Acylgruppe oder $R_4$,
$R_2$ Methyl, Ethyl, Isopropyl oder sek.Butyl und
$R_3$ unsubstituierte oder substituierte gerad- oder verzweigtkettige $C_1$-$C_{18}$-Alkyl-Gruppen, unsubstituierte oder substituierte cycloaliphatische Gruppen mit 3 bis 10 Kohlenstoffatomen, unsubstituierte oder substituierte $C_2$-$C_6$-Alkenyl-Gruppen, unsubstituierte oder substituierte $C_2$-$C_6$-Alkinyl-Gruppen, unsubstituiertes oder substituiertes Phenyl oder unsubstituiertes oder substituiertes Benzyl bedeuten.
Die Wirkstoffe besitzen vorteilhafte pestizide Eigenschaften. Sie eignen sich insbesondere zur Bekämpfung von Schädlingen in der Land- und Vorratswirtschaft.



ACTORUM AG

0245209

BEZEICHNUNG GEÄNDERT
siehe Titelseite

CIBA-GEIGY AG
Basel (Schweiz)

5-15866/=

# Pestizide

Die vorliegende Erfindung betrifft neue 13ß-Oxycarbonyloxy-Milbemycin-Derivate der Formel I, ihre Herstellung und ihre Verwendung zur Bekämpfung von Schädlingen, ferner Schädlingsbekämpfungsmittel, die mindestens eine dieser Verbindungen als Wirkstoff enthalten.

Die neuen Verbindungen entsprechen der allgemeinen Formel I

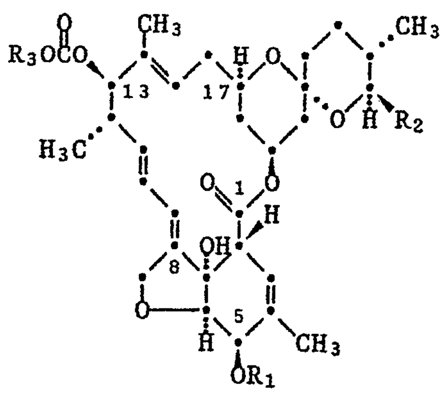

(I)

in welcher

$R_1$ Wasserstoff, eine Silyl- oder Acylgruppe oder $R_4$,

$R_2$ Methyl, Ethyl, Isopropyl oder sek.Butyl und

$R_3$ unsubstituierte oder substituierte gerad- oder verzweigtkettige $C_1$-$C_{18}$-Alkyl-Gruppen, unsubstituierte oder substituierte cycloaliphatische Gruppen mit 3 bis 10 Kohlenstoffatomen, unsubstituierte oder substituierte $C_2$-$C_6$-Alkenyl-Gruppen, unsubstituierte oder

substituierte $C_2$-$C_6$-Alkinyl-Gruppen, unsubstituiertes oder substituiertes Phenyl oder unsubstituiertes oder substituiertes Benzyl bedeuten.

Unter den vorgenannten Bedeutungen von $R_3$ sind als bevorzugt anzusehen: unsubstituiertes oder substituiertes $C_1$-$C_8$-Alkyl, unsubstituiertes oder substituiertes $C_3$-$C_6$-Cycloalkyl, unsubstituiertes oder substituiertes $C_2$-$C_6$-Alkenyl und unsubstituiertes oder substituiertes $C_2$-$C_6$-Alkinyl und von diesen vor allem die unsubstituierten Kohlenwasserstoffgruppen.

Als Substituenten der unter $R_3$ aufgeführten Alkylgruppen, cycloaliphatischen Gruppen, Alkenyl- und Alkinylgruppen kommen vorzugsweise Halogenatome, Hydroxy-, Alkoxy- und Acyloxygruppen in Betracht, wobei im allgemeinen nicht mehr als 7 solcher Substituenten vorliegen. Von den vier vorgenannten Kohlenwasserstoffgruppen sind insbesondere solche erwähnenswert, welche i) 1 bis 7 Halogenatome, vorzugsweise Halogenatome aus der Gruppe bestehend aus Chlor-, Fluor- und Bromatomen, ii) 1 bis 6 Hydroxygruppen, iii) 1-6 $C_1$-$C_6$-Alkoxygruppen oder iv) 1-6 Acyloxygruppen, vorzugsweise $C_1$-$C_5$-Alkylcarbonyloxygruppen, enthalten. Ferner kommen für $R_3$ als Substituenten der vorgenannten vier Kohlenwasserstoffreste auch unsubstituierte oder substituierte Oxacycloalkylgruppen in Frage, insbesondere monocyclische, 5- oder 6-gliedrige Oxacycloalkylgruppen mit 1 oder 2 Sauerstoffatomen. Hiervon bevorzugt sind (Oxacycloalkyl)-alkylgruppen, vor allem solche, in denen das Oxacycloalkyl ein- oder mehrfach durch $C_1$-$C_3$-Alkyl, vorzugsweise Methyl, substituiert und über $C_1$-$C_4$-Alkyl, vorzugsweise Methyl, gebunden ist. Als Beispiele für (Oxacycloalkyl)-alkylgruppen sind Tetrahydropyran-2-ylmethyl, 1,3-Dioxolan-4-ylmethyl, und insbesondere (2,2-Dimethyl-1,3-dioxolan-4-yl)-methyl zu nennen. Wenn $R_3$ eine Phenyl- oder Benzylgruppe bedeutet, kann diese durch 1 bis 3 Substituenten substituiert sein, ausgewählt aus der Gruppe bestehend aus Halogenatomen, $C_1$-$C_6$-Alkyl, $C_1$-$C_6$-Alkoxy, $C_1$-$C_4$-Alkylthio, Nitro oder

$-COR_5$, worin $R_5$ für Hydroxyl, $C_1$-$C_4$-Alkoxy oder $-NR_6R_7$ steht, worin $R_6$ und $R_7$ unabhängig voneinander Wasserstoff oder $C_1$-$C_4$-Alkyl bedeuten.

Unter dem Begriff Alkyl als Substituent oder Teil eines Substituenten sind je nach Zahl der angegebenen Kohlenstoffatome beispielsweise folgende Gruppen zu verstehen: Methyl, Ethyl, Propyl, Butyl, Pentyl, Hexyl, Heptyl, Octyl, Nonyl oder Decyl, sowie die Isomeren, wie beispielsweise Isopropyl, Isobutyl, tert.-Butyl, Isopentyl oder 2-Ethyl-n-hexyl. Haloalkyl steht für einen einfach bis perhalogenierten Alkylsubstituenten, insbesondere für $C_1$-$C_4$-Alkyl mit 1 bis 5 Halogenatomen aus der Gruppe bestehend aus Chlor-, Fluor- und Bromatomen, wie beispielsweise $CHCl_2$, $CHF_2$, $CH_2Cl$, $CCl_3$, $CH_2CCl_3$, $C(CH_3)_2CCl_3$, $CF_3$, $CH_2F$, $CH_2CH_2Br$, $CH_2CH_2Cl$, $CHBr_2$ und $CH_2CCl_2CF_3$. Im Rahmen der vorliegenden Erfindung steht Halogen für Fluor, Chlor, Brom oder Jod, vorzugsweise für Fluor, Chlor und Brom. Als durch Hydroxyl substituierte Alkylgruppen kommen vorzugsweise solche mit 1 bis 4 Kohlenstoffatomen und 1 oder 2 Hydroxygruppen in Betracht, wie beispielsweise $CH_2OH$, $CH_2CH(CH_3)CH_2OH$, $CH_2CH(OH)CH_2OH$ oder $CH_2CH_2CH_2CH_2OH$. Von den durch Alkoxy substituierten Alkylgruppen sind diejenigen besonders erwähnenswert, welche insgesamt 2 bis 8 Kohlenstoffatome und 1 oder 2 Sauerstoffatome enthalten, wie beispielsweise $CH_2OCH_3$, $CH_2CH_2OCH_3$, $CH_2CH(CH_3)OCH_3$, $CH_2OC_2H_5$, $CH_2C(OCH_3)_2CH_3$, $CH_2CH(OCH_3)CH_2OCH_3$, $C(CH_3)_2OCH_3$, $CH_2O$iso$C_3H_7$ oder $CH_2CH_2CH_2OCH_3$. Als durch Acyloxy substituierte Alkylgruppen kommen bevorzugt solche in Frage, welche insgesamt 2 bis 8 Kohlenstoffatome und nicht mehr als eine Carbonyloxygruppe aufweisen, wie beispielsweise $CH_2OCHO$, $CH_2CH_2OCHO$, $CH_2OC(O)CH_2CH_3$, $CH_2CH_2CH(OC(O)CH_3)CH_3$, $CH_2CH_2CH_2OC(O)CH_3$ und insbesondere $CH_2OC(O)CH_3$.

Als cycloaliphatische Gruppen kommen mono- bis tetracyclische Gruppen in Betracht, wie beispielsweise Cyclopropyl, Cyclobutyl, Cyclopentyl, Cyclohexyl, Decalin, Hydrindan, Bicycloheptan, Bicyclooctan, Norbornan, Bornan oder Adamantyl. Diese cycloaliphatischen Gruppen sind vorzugsweise unsubstituiert oder durch $C_1$-$C_4$-

Alkyl ein- oder mehrfach substituiert. Bevorzugt sind gesättigte, monocyclische, unsubstituierte oder durch $C_1$-$C_3$-Alkylgruppen ein- bis vierfach substituierte cycloaliphatische Gruppen mit 3 bis 6 Kohlenstoffatomen, wie beispielsweise Cyclopentyl, Cyclohexyl, 2-Methylcyclopentyl, 3,3-Dimethylcyclopentyl, 4-Methylcyclohexyl, 3,5-Dimethylcyclohexyl, 3,3,4,5-Tetramethylcyclohexyl, 4-(1-Methylethyl)-cyclohexyl oder 2-(1-Methylethyl)-5-methylcyclohexyl.

Alkenyl steht für einen mindestens durch eine C=C-Doppelbindung charakterisierten aliphatischen, acyclischen Kohlenwasserstoffrest, wie beispielsweise für Vinyl, Propenyl-(1), Allyl, Butenyl-(1), Butenyl-(2) oder Butenyl-(3).

Alkinyl steht für eine gerade oder verzweigte Kohlenstoffkette, die durch mindestens eine C≡C Dreifachbindung charakterisiert ist. Typische Vertreter sind beispielsweise Ethinyl, Propionyl-(1), Propargyl oder Butinyl-(1).

Substituiertes Phenyl oder Benzyl ist vorzugsweise durch 1 bis 3 Substituenten aus der Gruppe bestehend aus Chlor, Brom, Fluor, $C_1$-$C_3$-Alkyl, insbesondere Methyl, $C_1$-$C_3$-Alkoxy, insbesondere Methoxy, $C_1$-$C_3$-Alkylthio, insbesondere Methylthio, Nitro oder $-COR_5$, worin $R_5$ für $C_1$-$C_4$-Alkoxy, insbesondere Methoxy, steht, ringsubstituiert. Beispiele hierfür sind 2,4-Dichlorphenyl, 2,4,5-Trichlorphenyl, 4-Bromphenyl, 2,4-Xylyl, 4-Nitrophenyl, 4-(Methoxycarbonyl)-phenyl, 4-Chlor-2-methylphenyl, 4-Methyl-2-methoxyphenyl, 2,4,6-Trimethylphenyl, 4-Methylthiophenyl, 3,4-Dichlorbenzyl, 3,5-Dimethylbenzyl, 4-Methoxybenzyl, 4-Nitrobenzyl und 4-(Methoxycarbonyl)-benzyl in Betracht.

Als Beispiele für $R_3$, die keine Limitierung darstellen, sind zu nennen: Methyl, Ethyl, n-Propyl, Isopropyl, n-Butyl, sek.Butyl, tert.Butyl, Isobutyl, 2-Methylbutyl, n-Pentyl, Isopentyl, Neopentyl, n-Hexyl, Isohexyl, n-Heptyl, 3-Ethylpentyl, n-Octyl, 2-Ethylhexyl, 2-Propylpentyl; Chlormethyl, Brommethyl, Dichlormethyl, Trichlormethyl, Trifluormethyl, 2-Chlorethyl, 2-Bromethyl, 2,2,2-Trichlorethyl, 2,2,2-Trifluorethyl, 1,1-Dimethyl-2,2,2-trichlorethyl, 3,3,3-Trifluorpropyl, 2,2-Dichlor-3,3,3-trifluorpropyl; Hydroxymethyl, 2-Hydroxyethyl, 3-Hydroxypropyl, 2,2-Dihydroxyethyl, 1-Hydroxymethyl-2-hydroxyethyl, 2,3-Dihydroxypropyl, 3,4-Dihydroxybutyl; Methoxymethyl, Propoxymethyl, 2-Methoxyethyl, 2,3-Dimethoxypropyl, 4-Ethoxybutyl; Acetoxymethyl, 2-(Acetoxy)-ethyl, 4-(Acetoxy)-butyl, Propionyloxymethyl, 3-(Propionyloxy)-propyl, Isobutyryloxymethyl, 2-(Valeryloxy)-ethyl; Tetrahydrofuran-2-ylmethyl, Tetrahydrofuran-2-ylpropyl, (3,4-Dimethyltetrahydrofuran-2-yl)-methyl, 1,3-Dioxolan-4-ylmethyl, (2,2,-Dimethyl-1,3-dioxolan-4-yl)-methyl, (2,2-Dimethyl-1,3-dioxolan-4-yl)-ethyl, (2,2,4,4-Tetramethyl-1,3-dioxolan-5-yl)-methyl, Tetrahydropyran-2-ylmethyl, (1,3-Dioxa-4-cyclohexyl)-methyl, (1,4-Dioxa-2-cyclohexyl)-methyl; Cyclopentyl, Cyclohexyl, 4-Methylcyclohexyl, 2,5-Dimethylcyclohexyl, 2,6-Dimethylcyclohexyl, 2-(1-Methylethyl)-5-methylcyclohexyl; Vinyl, Allyl, Isopropenyl, 2-Butenyl; Phenyl, 4-Chlorphenyl, 2,4,5-Trichlorphenyl, 4-Nitrophenyl, 3,5-Dinitrophenyl, 3-Methoxycarbonylphenyl, 4-Methoxycarbonylphenyl; Benzyl, 3-Nitrobenzyl und 4-Nitrobenzyl.

Verbindungen der Formel I, worin $R_1$ Wasserstoff bedeutet, sind bevorzugt. Acyl- und Silylgruppen als $R_1$ sind im allgemeinen als Schutzgruppen aufzufassen. Geeignete Acylgruppen sind beispielsweise die Reste $R_8$-C(O)-, wobei $R_8$ für $C_1$-$C_{10}$-Alkyl, $C_1$-$C_{10}$-Haloalkyl oder einen unsubstituierten oder durch Substituenten der Gruppe bestehend aus Halogen, $C_1$-$C_3$-Alkyl, $C_1$-$C_3$-Haloalkyl, $C_1$-$C_3$-Alkoxy, $C_1$-$C_3$-Haloalkoxy, Cyano und Nitro substituierten Rest aus der Gruppe Phenyl und Benzyl steht und vorzugsweise $C_1$-$C_6$-Alkyl, $C_1$-$C_6$-Haloalkyl oder unsubstituiertes oder durch Halogen, $C_1$-$C_3$-Alkyl, $CF_3$ oder Nitro substituiertes Phenyl bedeutet. Besonders bevorzugt ist die Acetylgruppe. Als Acylverbindung ist beispielsweise 5-O-Acetyl-13β-(methoxycarbonyloxy)-milbemycin $A_4$ zu nennen. Als geeignete Silylgruppen für $R_1$ kommt der Rest -Si($R_9$)($R_{10}$)($R_{11}$) in Frage, wobei $R_9$, $R_{10}$ und $R_{11}$ vorzugsweise unabhängig voneinander für $C_1$-$C_6$-Alkyl, Benzyl oder Phenyl stehen und beispielsweise eine der Gruppen

Trimethylsilyl, Thexyldimethylsilyl (Thexyl=1,1,2-Trimethylpropyl), Diphenyl-tert.butylsilyl, bis(Isopropyl)methylsilyl oder Triphenylsilyl und vorzugsweise tert.-Butyldimethylsilyl bilden.

Für $R_4$ kommen unsubstituiertes oder substituiertes Benzyl oder unsubstituiertes oder substituiertes $C_1$-$C_4$-Alkyl in Betracht, wobei als Substituenten die unter $R_3$ für die entsprechenden Reste angegebenen Substituenten zu nennen sind und für den Alkylrest zusätzlich auch $C_1$-$C_4$-Alkoxy-$C_1$-$C_4$-alkoxy als Substituent in Frage kommt. Besonders hervorzuheben für $R_4$ sind Benzyl und Methoxyethoxymethyl.

Verbindungen, worin $R_2$ sek.Butyl darstellt, sollen hier und im folgenden gleichfalls zu den Milbemycin-Derivaten gerechnet werden, obwohl sie nach der üblichen Systematik von Avermectin-Derivaten abgeleitet sind. Avermectin-Aglykone (mit einer OH-Gruppe in 13α-Position) lassen sich jedoch gemäss US-PS 4,173,571 in Milbemycin-Homologe überführen.

In natürlich vorkommenden Milbemycinen ($R_1$=H; $R_2$=$CH_3$, $C_2H_5$ oder iso-$C_3H_7$) ist die 13-Position anstelle der Carbonyldioxygruppe der erfindungsgemässen Verbindungen der Formel I ausschliesslich mit Wasserstoff besetzt, wie die nachstehende Formel zeigt:

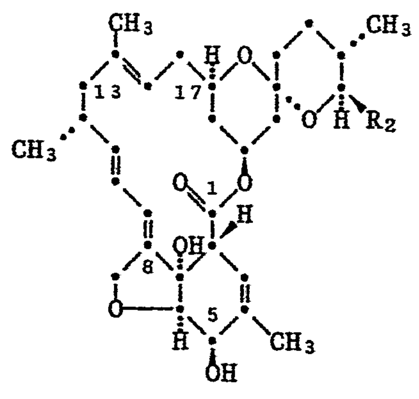

| | | |
|---|---|---|
| $R_2$ | = $CH_3$ | Milbemycin $A_3$ |
| $R_2$ | = $C_2H_5$ | Milbemycin $A_4$ |
| $R_2$ | = iso$C_3H_7$ | Milbemycin D |
| $R_2$ | = sec.$C_4H_9$ | 13-Deoxi-22,23-dihydro-C-076-Bla-aglycon. |

Bei Avermectinen dagegen steht in der 13-Position ein α-L-Oleandrosyl-α-L-oleandrose-Rest, der über Sauerstoff in α-Konfiguration mit dem Makrolid-Molekül verknüpft ist. Avermectine unterscheiden sich strukturell ausserdem durch eine 23-OH-Gruppe oder $\Delta^{22,23}$-Doppelbindung und in der Regel durch einen Substituenten $R_2$ = sek.$C_4H_9$ von den Milbemycinen. Durch Hydrolyse des Zucker-Restes der Avermectine gelangt man leicht zu den entsprechenden Avermectin-Aglykonen, die eine in Nachbarstellung zu einer C=C-Doppelbindung befindliche 13α-Hydroxy-Gruppe besitzen. Die Avermectin-Aglykone sind, wie vorstehend angegeben, in die Milbemycin-Homologen umwandelbar. Bei den Milbemycin-Derivaten der vorliegenden Anmeldung liegt die $\Delta^{22,23}$-Doppelbindung stets in hydrierter Form vor und der Substituent in 13-Position ist immer β-ständig.

Verbindungen der Formel I, worin $R_1$ eine andere Bedeutung als Wasserstoff hat, lassen sich durch einfache, beispielsweise hydrolytische Abspaltung des Substituenten $R_1$ in die hochaktiven freien 5-Hydroxy-derivate ($R_1$=H) überführen und haben somit Zwischenprodukte-Charakter. Im übrigen wird der biologische Wert dieser Verbindungen durch die Schutzgruppe nicht gemindert.

Folgende Untergruppen von Verbindungen der Formel I sind auf Grund ihrer ausgeprägten Wirkung gegen Schädlinge besonders bevorzugt:

Gruppe Ia: Verbindungen der Formel I, worin $R_1$ Wasserstoff oder tert.-Butyldimethylsilyl, vorzugsweise Wasserstoff, darstellt, $R_2$ für Methyl, Ethyl, Isopropyl oder sek.Butyl steht und $R_3$ folgende Bedeutungen hat:

Alkyl mit 1 bis 10, vorzugsweise 1 bis 8 Kohlenstoffatomen, welches unsubstituiert oder durch 1 bis 6 Halogenatome, vorzugsweise aus der Gruppe bestehend aus Chlor-, Fluor- und Bromatomen, substituiert ist, oder durch Hydroxyl oder $C_1$-$C_4$-Alkoxy, vorzugsweise Methoxy, mono- bis trisubstituiert ist, oder durch ein monocyclisches, 5-oder 6-gliedriges, 1 oder 2 Sauerstoffatome enthaltendes, unsubsti-

tuiertes oder durch $C_1$-$C_3$-Alkyl, vorzugsweise Methyl, ein- bis vierfach substituiertes Oxacycloalkyl monosubstituiert ist; $C_3$-$C_6$-Cycloalkyl, bevorzugt Cyclohexyl, welches unsubstituiert oder durch $C_1$-$C_3$-Alkyl mono- bis trisubstituiert ist; Alkenyl mit 2 bis 4 Kohlenstoffatomen; Phenyl, welches unsubstituiert oder durch Halogen, vorzugsweise Chlor, mono- bis trisubstituiert ist oder durch $C_1$-$C_3$-Alkoxycarbonyl, vorzugsweise Methoxycarbonyl, oder Nitro mono- oder disubstituiert ist; oder Benzyl, welches unsubstituiert oder durch Nitro mono- oder disubstituiert ist;

Gruppe Ib: Verbindungen der Formel I, worin $R_1$ Wasserstoff oder tert.-Butyldimethylsilyl, vorzugsweise Wasserstoff darstellt, $R_2$ für Methyl, Ethyl, Isopropyl oder sek.Butyl, bevorzugt Ethyl oder Isopropyl, steht und $R_3$ folgende Bedeutungen hat:
Alkyl mit 1 bis 8 Kohlenstoffatomen; Alkyl mit 1 bis 4 Kohlenstoffatomen, welches durch 1 bis 5 Halogenatome, vorzugsweise aus der Gruppe bestehend aus Chlor-, Fluor- und Bromatomen, substituiert ist oder durch Hydroxyl oder $C_1$-$C_3$-Alkoxy, vorzugsweise Methoxy, mono- oder disubstituiert ist; Methyl, welches durch Oxacycloalkyl, ausgewählt aus der Gruppe bestehend aus Tetrahydrofuranyl, 1,3-Dioxolanyl und Tetrahydropyranyl, monosubstituiert ist, wobei das Oxacycloalkyl unsubstituiert oder durch Methyl ein- bis vierfach substituiert ist; Cyclohexyl, welches unsubstituiert oder durch $C_1$-$C_3$-Alkyl mono- oder disubstituiert ist; Alkenyl mit 2 bis 4 Kohlenstoffatomen; Phenyl, welches unsubstituiert oder durch Chlor mono- bis trisubstituiert ist, oder durch Methoxycarbonyl oder Nitro mono- oder disubstituiert ist; oder Benzyl, welches unsubstituiert oder durch Nitro mono- oder disubstituiert ist.

Gruppe Ic: Verbindungen der Formel I, worin $R_1$ Wasserstoff oder tert.-Butyldimethylsilyl, vorzugsweise Wasserstoff, darstellt, $R_2$ für Methyl, Ethyl oder Isopropyl steht und $R_3$ folgende Bedeutungen hat:
Alkyl mit 1 bis 3 Kohlenstoffatomen, welches unsubstituiert oder durch Chlor mono- bis trisubstituiert ist, oder durch Hydroxy mono- oder disubstituiert ist oder durch 1,3-Dioxolanyl, welches

unsubstituiert oder durch Methyl ein- bis vierfach, vorzugsweise in 2-Position disubstituiert ist, monosubstituiert ist; Cyclohexyl; oder $C_2$-$C_4$-Alkenyl, vorzugsweise Vinyl oder Allyl;

Gruppe Id: Verbindungen der Formel I, worin $R_1$ Wasserstoff oder tert.Butyldimethylsilyl, vorzugsweise Wasserstoff, darstellt, $R_2$ Methyl, Ethyl oder Isopropyl und $R_3$ Methyl, Vinyl oder Allyl bedeuten;

Gruppe Ie: Verbindungen der Formel I, worin $R_1$ Wasserstoff, $R_2$ Methyl, Ethyl oder Isopropyl und $R_3$ Methyl, Ethyl, 2,2,2-Trichlorethyl, 2,3-Dihydroxypropyl, 2,2-Dimethyl-1,3-dioxolan-4-ylmethyl oder Cyclohexyl bedeuten;

Gruppe If: Verbindungen der Formel I, worin $R_1$ tert.Butyldimethylsilyl, $R_2$ Methyl, Ethyl oder Isopropyl und $R_3$ Methyl oder Cyclohexyl bedeuten;

Gruppe Ig: Verbindungen der Formel I, worin $R_1$ Wasserstoff, $R_2$ Methyl, Ethyl oder Isopropyl und $R_3$ Methyl, Ethyl, 1,1-Dimethyl-2,2,2-trichlorethyl, (2,2-Dimethyl-1,3-dioxolan-4-yl)-methyl oder Cyclohexyl bedeuten, mit den Einzelverbindungen
13β-(Methoxycarbonyloxy)-milbemycin $A_3$ (Nr. 1.1),
13β-(Methoxycarbonyloxy)-milbemycin $A_4$ (Nr. 1.2),
13β-(Methoxycarbonyloxy)-milbemycin D (Nr. 1.3),
13β-(Ethoxycarbonyloxy)-milbemycin $A_3$ (Nr. 1.5),
13β-(Ethoxycarbonyloxy)-milbemycin $A_4$ (Nr. 1.6),
13β-(Ethoxycarbonyloxy)-milbemycin D (Nr. 1.7),
13β-[(1,1-Dimethyl-2,2,2-trichlorethoxy)-carbonyloxy]-milbemycin $A_3$ (Nr. 1.33),
13β-[(1,1-Dimethyl-2,2,2-trichlorethoxy)-carbonyloxy]-milbemycin $A_4$ (Nr. 1.34),
13β-[(1,1-Dimethyl-2,2,2-trichlorethoxy)-carbonyloxy]-milbemycin D (Nr. 1.35),
13β-[(2,2-Dimethyl-1,3-dioxolan-4-yl)-methoxycarbonyloxy]-milbemycin $A_3$ (Nr. 1.65),

13β-[(2,2-Dimethyl-1,3-dioxolan-4-yl)-methoxycarbonyloxy]-milbemycin $A_4$ (Nr. 1.66),
13β-[(2,2-Dimethyl-1,3-dioxolan-4-yl)-methoxycarbonyloxy]-milbemycin D (Nr. 1.67),
13β-(Cyclohexyloxycarbonyloxy)-milbemycin $A_3$ (Nr. 1.81),
13β-(Cyclohexyloxycarbonyloxy)-milbemycin $A_4$ (Nr. 1.82), und
13β-(Cyclohexyloxycarbonyloxy)-milbemycin D (Nr. 1.83).

Gruppe Ih: Verbindungen der Formel I, worin $R_1$ tert.Butyldimethylsilyl, $R_2$ Methyl, Ethyl oder Isopropyl und $R_3$ Methyl, Ethyl, 1,1-Dimethyl-2,2,2-trichlorethyl, (2,2-Dimethyl-1,3-dioxolan-4-yl)-methyl oder Cyclohexyl bedeuten, mit den Einzelverbindungen
5-O-tert.-Butyldimethylsilyl-13β-(methoxycarbonyloxy)-milbemycin $A_3$ (Nr. 2.1),
5-O-tert.-Butyldimethylsilyl-13β-(methoxycarbonyloxy)-milbemycin $A_4$ (Nr. 2.2),
5-O-tert.-Butyldimethylsilyl-13β-(methoxycarbonyloxy)-milbemycin D (Nr. 2.3),
5-O-tert.-Butyldimethylsilyl-13β-(ethoxycarbonyloxy)-milbemycin $A_3$ (Nr. 2.5),
5-O-tert.-Butyldimethylsilyl-13β-(ethoxycarbonyloxy)-milbemycin $A_4$ (Nr. 2.6),
5-O-tert.-Butyldimethylsilyl-13β-(ethoxycarbonyloxy)-milbemycin D (Nr. 2.7),
5-O-tert.-Butyldimethylsilyl-13β-[(1,1-dimethyl-2,2,2-trichlor-ethoxy)-carbonyloxy]-milbemycin $A_3$ (Nr. 2.33),
5-O-tert.-Butyldimethylsilyl-13β-[(1,1-dimethyl-2,2,2-trichlor-ethoxy)-carbonyloxy]-milbemycin $A_4$ (Nr. 2.34),
5-O-tert.-Butyldimethylsilyl-13β-[(1,1-dimethyl-2,2,2-trichlor-ethoxy)-carbonyloxy]-milbemycin D (Nr. 2.35),
5-O-tert.-Butyldimethylsilyl-13β-[(2,2-dimethyl-1,3-dioxolan-4-yl)-methoxycarbonyloxy]-milbemycin $A_3$ (Nr. 2.65),
5-O-tert.-Butyldimethylsilyl-13β-[(2,2-dimethyl-1,3-dioxolan-4-yl)-methoxycarbonyloxy]-milbemycin $A_4$ (Nr. 2.66),
5-O-tert.-Butyldimethylsilyl-13β-[(2,2-dimethyl-1,3-dioxolan-4-yl)-methoxycarbonyloxy]-milbemycin D (Nr. 2.67),

5-O-tert.-Butyldimethylsilyl-13β-(cyclohexyloxycarbonyloxy)-milbemycin $A_3$ (Nr. 2.81),
5-O-tert.-Butyldimethylsilyl-13β-(cyclohexyloxycarbonyloxy)-milbemycin $A_4$ (Nr. 2.82), und
5-O-tert.-Butyldimethylsilyl-13β-(cyclohexyloxycarbonyloxy)-milbemycin D (Nr. 2.83).

Gruppe Ii: Verbindungen der Formel I, worin $R_1$ Acetyl, $R_2$ Methyl, Ethyl oder Isopropyl und $R_3$ Methyl, Ethyl, 1,1-Dimethyl-2,2,2-trichlorethyl, (2,2-Dimethyl-1,3-dioxolan-4-yl)-methyl oder Cyclohexyl bedeuten, mit den Einzelverbindungen
5-O-Acetyl-13β-(methoxycarbonyloxy)-milbemycin $A_3$ (Nr. 3.1),
5-O-Acetyl-13β-(methoxycarbonyloxy)-milbemycin $A_4$ (Nr. 3.2),
5-O-Acetyl-13β-(methoxycarbonyloxy)-milbemycin D (Nr. 3.3),
5-O-Acetyl-13β-(ethoxycarbonyloxy)-milbemycin $A_3$ (Nr. 3.5),
5-O-Acetyl-13β-(ethoxycarbonyloxy)-milbemycin $A_4$ (Nr. 3.6),
5-O-Acetyl-13β-(ethoxycarbonyloxy)-milbemycin D (Nr. 3.7),
5-O-Acetyl-13β-[(1,1-dimethyl-2,2,2-trichlorethoxy)-carbonyloxy]-milbemycin $A_3$ (Nr. 3.33),
5-O-Acetyl-13β-[(1,1-dimethyl-2,2,2-trichlorethoxy)-carbonyloxy]-milbemycin $A_4$ (Nr. 3.34),
5-O-Acetyl-13β-[(1,1-dimethyl-2,2,2-trichlorethoxy)-carbonyloxy]-milbemycin D (Nr. 3.35),
5-O-Acetyl-13β-[(2,2-dimethyl-1,3-dioxolan-4-yl)-methoxycarbonyl-oxy]-milbemycin $A_3$ (Nr. 3.65),
5-O-Acetyl-13β-[(2,2-dimethyl-1,3-dioxolan-4-yl)-methoxycarbonyl-oxy]-milbemycin $A_4$ (Nr. 3.66),
5-O-Acetyl-13β-[(2,2-dimethyl-1,3-dioxolan-4-yl)-methoxycarbonyl-oxy]-milbemycin D (Nr. 3.67),
5-O-Acetyl-13β-(cyclohexyloxycarbonyloxy)-milbemycin $A_3$ (Nr. 3.81),
5-O-Acetyl-13β-(cyclohexyloxycarbonyloxy)-milbemycin $A_4$ (Nr. 3.82), und
5-O-Acetyl-13β-(cyclohexyloxycarbonyloxy)-milbemycin D (Nr. 3.83).

Gruppe Ik: Verbindungen der Formel I, worin $R_1$ Wasserstoff oder tert.-Butyldimethylsilyl, $R_2$ Methyl, Ethyl oder Isopropyl und $R_3$ Methyl, Vinyl, Allyl oder Cyclohexyl bedeuten oder zusätzlich, wenn $R_1$ Wasserstoff bedeutet, $R_3$ auch für Ethyl, 2,2,2-Trichlorethyl, 2,2-Dimethyl-1,3-dioxolan-4-ylmethyl oder 2,3-Dihydroxy-n-propyl steht, mit den bevorzugten Einzelverbindungen
13β-(Methoxycarbonyloxy)-milbemycin $A_4$ (Nr. 1.2),
13β-(Methoxycarbonyloxy)-milbemycin D (Nr. 1.3),
13β-(Ethoxycarbonyloxy)-milbemycin $A_4$ (Nr. 1.6),
13β-[(2,2,2-Trichlorethoxy)-carbonyloxy]-milbemycin $A_4$ (Nr. 1.26),
13β-(Vinyloxycarbonyloxy)-milbemycin D (Nr. 1.39),
13β-(Allyoxycarbonyloxy)-milbemycin D (Nr. 1.43),
13β-[(2,2-Dimethyl-1,3-dioxolan-4-yl)-methoxycarbonyloxy]-milbemycin $A_4$ (Nr. 1.66),
13β-[(2,3-Dihydroxypropoxy)-carbonyloxy]-milbemycin $A_4$ (Nr. 1.70),
13β-(Cyclohexyloxycarbonyloxy)-milbemycin $A_4$ (Nr. 1.82),
5-O-tert.-Butyldimethylsilyl-13β-(methoxycarbonyloxy)-milbemycin $A_4$ (Nr. 2.2),
5-O-tert.-Butyldimethylsilyl-13β-(methoxycarbonyloxy)-milbemycin D (Nr. 2.3),
5-O-tert.-Butyldimethylsilyl-13β-(vinyloxycarbonyloxy)-milbemycin D (Nr. 2.39),
5-O-tert.-Butyldimethylsilyl-13β-(allyloxycarbonyloxy)-milbemycin D (Nr. 2.43) und
5-O-tert.-Butyldimethylsilyl-13β-(cyclohexyloxycarbonyloxy)-milbemycin $A_4$ (Nr. 2.82).

Bevorzugt sind die Gruppen g) und h) nebst den dazu angeführten Einzelverbindungen, insbesondere Gruppe g) und Einzelverbindungen, welche im Umfang dieser Gruppe liegen.

Bemerkenswerte Verbindungen der Formel I sind ferner 5-O-Acetyl-13β-(methoxycarbonyloxy)-milbemycin $A_4$ (Nr. 3.2) und 13β-(Tetrahydrofuran-2-ylmethoxycarbonyloxy)-milbemycin $A_4$ (Nr. 1.102).

Die vorliegende Erfindung betrifft auch Herstellungsverfahren, die es erlauben, in der 13-Position von Milbemycin- oder 13-Deoxi-22,23-dihydro-Avermectinaglykon-Derivaten eine β-Oxycarbonyloxy-Gruppe gezielt einzuführen und damit zu den hochwirksamen, neuen Parasitiziden und Insektiziden der Formel I zu gelangen, die ihrerseits auch für weitere Derivatisierungen verwendet werden können. Die erfindungsgemässe Herstellung der Verbindungen der Formel I lässt sich im Einzelnen wie folgt durchführen:

A. Eine Verbindung der Formel II

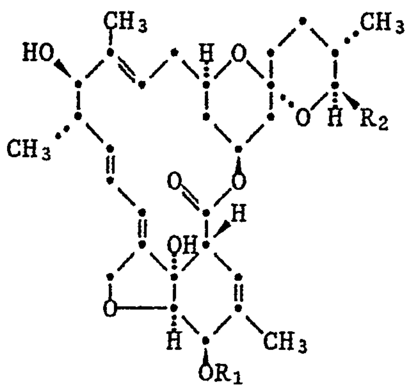

(II),

worin $R_1$ und $R_2$ die unter Formel I angegebenen Bedeutungen besitzen, wird mit einer zur Einführung einer 13β-Oxycarbonyloxygruppe $R_3O-C(O)-O-$ geeigneten Verbindung umgesetzt und danach, sofern ein Produkt mit einer freien 5-Hydroxy-Gruppe erwünscht ist, wird der Substituent $R_1$ hydrolytisch abgespalten.

Zur Einführung der 13β-Oxycarbonyloxy-Gruppe $R_3O-C(O)-O-$ eignen sich insbesondere Chlorameisensäureester der Formel (III)

$$R_3O-C(O)Cl \qquad (III)$$

worin $R_3$ die vorstehend für Formel I angegebene Bedeutung hat.

Als Beispiele für geeignete Verbindungen der Formel III im Rahmen vorliegender Erfindung seien genannt: Chlorameisensäuremethylester, -ethylester, -vinylester, -allylester, -benzylester, -bromethyl-

ester, -isobutylester, -(4-methoxycarbonylphenyl)-ester, 4-nitrobenzylester, -4-nitrophenylester, -phenylester, -2,2,2-trichlorethylester und -2,2,2-trichlor-tert.-butylester.

Die Umsetzung erfolgt vorzugsweise in einem inerten, hydroxylgruppenfreien Lösungsmittel in Anwesenheit einer organischen Base, wie z.B. Pyridin, 4-Dimethylaminopyridin, Lutidin, Collidin, Trialkylamin, N-Dialkylanilin, oder bicyclische, nicht nucleophile Basen wie z.B. 1,4-Diazabicyclo[2.2.2]octan (DABCO), 1,5-Diazabicyclo[4.3.0]-non-5-en (DBN) oder 1,8-Diazabicyclo[5.4.0]undec-7-en (1,5-5) (DBU). Die Reaktion wird im allgemeinen bei Temperaturen von -30° bis +70°C, vorzugsweise von -10° bis +50°C durchgeführt. Man arbeitet dabei zweckmässigerweise in Gegenwart eines reaktionsinerten Lösungsmittels oder Lösungsmittelgemisches. Es eignen sich hierfür z.B. aliphatische und aromatische Kohlenwasserstoffe wie Benzol, Toluol, Xylole, Petrolether, Hexan; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; Ester wie Ethylacetat (Essigsäureethylester), Propylacetat oder Butylacetat; Ketone wie Aceton, Diethylketon, Methylethylketon; Verbindungen wie Dimethylsulfoxid (DMSO), Dimethylformamid (DMF) und Gemische solcher Lösungsmittel untereinander. Man kann die Reaktion aber auch im Ueberschuss einer der oben genannten Basen durchführen.

B. Eine Verbindung der Formel IV,

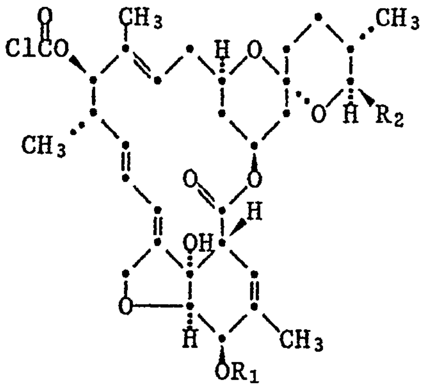

(IV),

worin $R_1$ und $R_2$ die unter Formel I angegebenen Bedeutungen haben, wird mit einer Verbindung der Formel V,

$R_3OH$ (V),

worin $R_3$ wie unter Formel I definiert ist, umgesetzt und, sofern ein Produkt mit einer freien 5-Hydroxy-Gruppe erwünscht ist, wird durch anschliessende hydrolytische Abspaltung der Substituent $R_1$ entfernt.

Die Reaktion wird vorzugsweise in einem der unter Variante A genannten Lösungsmittel und bevorzugt in Gegenwart einer der dort genannten Basen bei Temperaturen von -50° bis +50°C, vorzugsweise -20° bis +30°C durchgeführt.

Die Verbindungen der Formel IV lassen sich aus den Verbindungen der Formel II durch Behandlung mit Phosgen ($COCl_2$) herstellen, wobei man vorteilhafterweise bei den unter Variante B angegebenen Bedingungen arbeitet.

Zur Herstellung von Verbindungen der Formel I werden bevorzugt solche Verbindungen der Formeln II und IV eingesetzt, bei denen $R_1$ eine andere Bedeutung als Wasserstoff hat.

Zur Herstellung von Verbindungen der Formel I, welche im Substituenten $R_3$ freie Hydroxylgruppen aufweisen, werden bevorzugt solche Verbindungen der Formeln III und V eingesetzt, bei denen die im

Substituenten $R_3$ vorliegenden Hydroxylgruppen durch in der organischen Chemie gebräuchliche Schutzgruppen geschützt sind. Geeignete Schutzgruppen sind beispielsweise die für $R_1$ unter Formel I aufgeführten Strukturelemente mit Ausnahme von Wasserstoff, insbesondere die für $R_1$ angeführten Silyl- und Acylgruppen.

Verbindungen der Formel I, welche im Substituenten $R_3$ eine geschützte Hydroxylgruppe aufweisen, lassen sich durch einfache, z.B. hydrolytische, Abspaltung der Schutzgruppe in die entsprechenden Verbindungen mit freier Hydroxylgruppe im Substituenten $R_3$ überführen. Dasselbe gilt sinngemäss für Verbindungen der Formel I mit mehreren Hydroxylgruppen im Substituenten $R_3$.

Die zur Gewinnung der erfindungsgemässen Verbindungen der Formel I mittels der hierin beschriebenen Verfahren benötigten Ausgangsverbindungen der Formel II werden erhalten durch Reaktion von Verbindungen der Formel VI

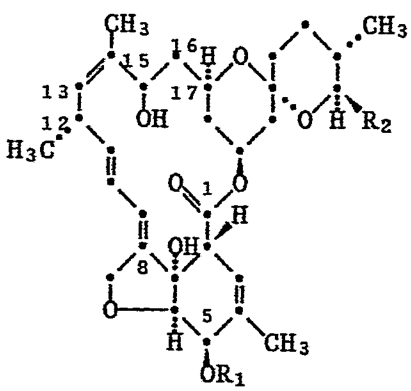

(VI)

worin $R_1$ und $R_2$ die unter Formel I angegebenen Bedeutungen haben, mit Chromat-, Halochromat- oder Dichromat-Ionen, insbesondere mit Pyridiniumdichromat [=$(Pyr)_2^+Cr_2O_7$] oder mit Pyridiniumchlorochromat [=$(Pyr)^+ClCrO_3$].

Es werden inerte wasserfreie, vorzugsweise polare Lösungsmittel, z.B. Dimethylformamid (=DMF) verwendet. Die Reaktion wird bei Temperaturen von -10°C bis +60°C, bevorzugt +10°C bis +40°C, durchgeführt.

Die Verbindungen der Formel VI [= $\Delta^{13,14}$-15-Hydroxy] lassen sich aus 14,15-Epoxy-Milbemycinen der Formel VII

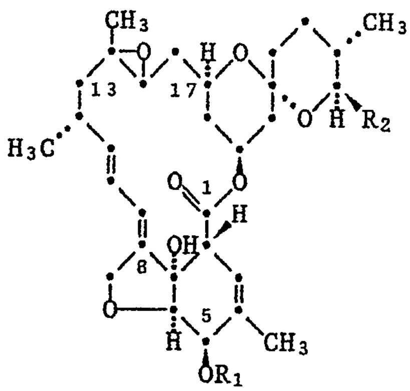

(VII)

worin $R_1$ und $R_2$ die für die Formel I genannten Bedeutungen haben, mit Hilfe des Komplex-Reagenzes $[HN_3]_m/[Al(Ethyl)_3]_n$, worin m und n unabhängig voneinander die Zahl 1 oder 2 oder einen Zahlenwert zwischen 1 und 2 darstellen, in inerten trockenen Lösungsmitteln im Temperaturbereich von -30° bis +10°C, vorzugsweise -20° bis -5°C, herstellen.

Als inerte Lösungsmittel kommen dafür vorzugsweise aliphatische und aromatische Kohlenwasserstoffe wie Benzol, Toluol, Xylol, Petrolether; Ether wie Diethylether, tert.Butylmethylether, Tetrahydrofuran, Dioxan oder Anisol in Frage.

Die Reaktion wird vorteilhaft unter Schutzgas, wie Stickstoff oder Argon, durchgeführt.

Stickstoffwasserstoffsäure $HN_3$ lässt sich in statu nascendi in den $[HN_3]_m/[Al(Et)_3]_n$-Komplex überführen, indem man im vorgesehenen trockenen Lösungsmittel oder Lösungsmittel-Gemisch Na-Azid suspendiert und daraus mit einer stärkeren Säure, beispielsweise $H_2SO_4$ (bevorzugt Oleum, um absolut trockene Reaktionsbedingungen zu gewährleisten), $HN_3$ in der Lösung in Freiheit setzt. $Al(Et)_3$ sollte in der Lösung bereits vorliegen oder kurz danach zugegeben werden. Die zur Reaktion vorgesehene Epoxy-Verbindung kann gleichfalls bereits vorliegen oder zu einem geeigneten Zeitpunkt zur Lösung dosiert werden.

0245209

Die für die Verbindungen VI verwendeten Ausgangsverbindungen der Formel VII lassen sich leicht herstellen durch Epoxydierung der aus der US-PS 3,950,360 bekanntgewordenen und ursprünglich als "Antibiotika B-41-A", später als "Milbemycin-A"-Verbindungen und der aus der US-PS 4,346,171 bekanngewordenen und als "B-41-D" oder "Milbemycin-D" bezeichneten Verbindungen sowie der aus der US-PS 4,173,571 und aus Tetrahedron Letters, Vol. 24, No. 48, pp. 5333-5336 (1983) bekanntgewordenen 13-Deoxy-22,23-dihydro-Avermectine ($R_2$ = sec.Butyl) der Formel

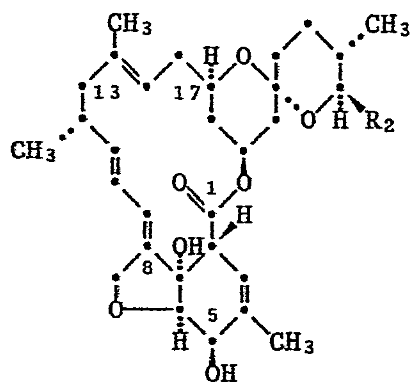

$R_2$ = $CH_3$ Milbemycin $A_3$
$R_2$ = $C_2H_5$ Milbemycin $A_4$
$R_2$ = iso$C_3H_7$ Milbemycin D
$R_2$ = sec.$C_4H_9$ 13-Deoxy-22,23-dihydro-C-076-Bla-aglycon.

Die Epoxidierung wird in einer Lösungsmittelphase im Temperaturbereich von -10° bis +20°C, vorzugsweise -5° bis +5°C, durchgeführt.

Die Epoxidierung wird mit Persäuren wie beispielsweise Peressigsäure, Trifluorperessigsäure, Perbenzoesäure oder Chlorperbenzoesäure durchgeführt.

Durch Acylierung oder Silylierung der 5-OH-Gruppe werden alle jene Derivate der Formeln I, II, IV, VI und VII hergestellt, bei denen $R_1$ für eine Acyl- oder Silylgruppe steht. Die Einführung der Acylgruppe erfolgt üblicherweise mit den entsprechenden Acylhalogeniden oder Acylanhydriden und wird vorzugsweise zur Einführung der eingangs

definierten $R_8C(O)$- Gruppe benutzt. Zur Silylierung verwendet man zweckmässigerweise ein Silan der Formel Y-Si($R_9$)($R_{10}$)($R_{11}$), worin $R_9$, $R_{10}$ und $R_{11}$ einen der eingangs genannten Reste darstellen, wobei der Begriff Acylhalogenid für Acylchlorid oder Acylbromid steht und wobei Y eine Silylabgangsgruppe bedeutet. Zu den Silylabgangsgruppen Y zählen beispielsweise Bromid, Chlorid, Cyanid, Azid, Acetamid, Trifluoracetat oder Trifluormethansulfonat. Diese Aufzählung stellt keine Limitierung dar, der Fachmann kennt weitere typische Silylabgangsgruppen.

5-O-Acylierungen und 5-O-Silylierungen werden in wasserfreiem Milieu, vorzugsweise in inerten Lösungsmitteln und besonders bevorzugt in aprotischen Lösungsmitteln durchgeführt. Die Reaktion läuft vorteilhaft im Temperaturbereich von 0° bis +80°C, bevorzugt +10° bis +40°C, ab. Vorzugsweise wird eine organische Base zugegeben. Es kommen als solche beispielsweise tertiäre Amine wie Triethylamin, Triethylendiamin, Triazol und bevorzugt Pyridin, Imidazol oder 1,8-Diazabicyclo[5.4.0]-undec-7-en (DBU) in Frage.

Die Verbindungen der Formel I, in denen $R_1$ für $R_4$ steht, sind auf an sich bekannte Weise durch Verätherung von Verbindungen der Formel I, in denen $R_1$ für Wasserstoff steht, erhältlich, wie beispielsweise durch Verätherung mit $R_4$-X, worin X für Chlor oder Brom steht.

Die Entfernung der Silyl- und Acylreste $R_1$ in der 5-Position geschieht durch selektive milde Hydrolyse ($\longrightarrow$ $R_1$=H) beispielsweise mit Arylsulfonsäure in alkoholischer Lösung oder nach einer anderen dem Fachmann geläufigen Methode. Entsprechendes gilt für Verbindungen, in denen $R_1$ für $R_4$ steht.

Das beschriebene Verfahren zur Herstellung der Verbindungen der Formel I ist in allen Teilschritten ein Bestandteil vorliegender Erfindung.

Die Verbindungen der Formel I eignen sich ausgezeichnet zur Bekämpfung von Schädlingen an Tieren und Pflanzen, darunter insbesondere von tierparasitären Ekto-Parasiten. Zu letzteren zählen unter der Ordnung Acarina insbesondere Schädlinge der Familien Ixodidae, Dermanyssidae, Sarcoptidae, Psoroptidae; die Ordnungen Mallophaga; Siphonaptera, Anoplura (z.B. Familie der Haemotopinidae); unter der Ordnung Diptera insbesondere Schädlinge der Familien Muscidae, Calliphoridae, Oestridae, Tabanidae, Hippoboscidae, Gastrophilidae.

Die Verbindungen I sind auch einsetzbar gegen Hygiene-Schädlinge, insbesondere der Ordnungen Diptera mit den Familien Sarcophagidae, Anophilidae, Culicidae; der Ordnung Orthoptera, der Ordnung Dictyoptera (z.B. Familie Blattidae) und der Ordnung Hymenoptera (z.B. Familie Formicidae).

Die Verbindungen I besitzen auch nachhaltige Wirksamkeit bei pflanzenparasitären Milben und Insekten. Bei Spinnmilben der Ordnung Acarina sind sie wirksam gegen Eier, Nymphen und Adulte von Tetranychidae (Tetranychus spp. und Panonychus spp.).

Hohe Aktivität besitzen sie bei den saugenden Insekten der Ordnung Homoptera, insbesondere gegen Schädlinge der Familien Aphididae, Delphacidae, Cicadellidae, Psyllidae, Coccidae, Diaspididae und Eriophydidae (z.B. die Rostmilbe auf Citrusfrüchten): der Ordnungen Hemiptera; Heteroptera und Thysanoptera; sowie bei den pflanzenfressenden Insekten der Ordnungen Lepidoptera; Coleoptera; Diptera und Orthoptera.

Sie sind ebenfalls als Bodeninsektizid gegen Schädlinge im Erdboden geeignet.

Die Verbindungen der Formel I sind daher gegen alle Entwicklungsstadien saugender und fressender Insekten an Kulturen wie Getreide, Baumwolle, Reis, Mais, Soja, Kartoffeln, Gemüse, Früchten, Tabak, Hopfen, Citrus, Avocados und anderen wirksam.

Die Verbindungen der Formel I sind auch wirksam gegen Pflanzen-Nematoden der Arten Meloidogyne, Heterodera, Pratylenchus, Ditylenchus, Radopholus, Rhizoglyphus und andere.

Ferner sind die Verbindungen gegen Helminthen wirksam, unter denen die endoparasitären Nematoden die Ursache schwerer Erkrankungen an Säugetieren und Geflügel sein können, z.B. an Schafen, Schweinen, Ziegen, Rindern, Pferden, Eseln, Hunden, Katzen, Meerschweinchen, Ziervögeln. Typische Nematoden dieser Indikation sind: Haemonchus, Trichostrongylus, Ostertagia, Nematodirus, Cooperia, Ascaris, Bunostomum, Oesophagostomum, Chabertia, Trichuris, Strongylus, Trichonema, Dictyocaulus, Capillaria, Heterakis, Toxocara, Ascaridia, Oxyuris, Ancylostoma, Uncinaria, Toxascaris und Parascaris. Der besondere Vorteil der Verbindungen der Formel I ist ihre Wirksamkeit gegen solche Parasiten, die gegen Wirkstoffe auf Benzimidazol-Basis resistent sind.

Gewisse Spezies der Arten Nematodirus, Cooperia und Oesophagostomum greifen den Intestinaltrakt des Wirtstiers an, während andere der Arten Haemonchus und Ostertagia im Magen und solche der Art Dictyocaulus im Lungengewebe parasitieren. Parasiten der Familien Filariidae und Setariidae finden sich im internen Zellgewebe und den Organen, z.B. dem Herzen, den Blutgefässen, den Lymphgefässen und dem subcutanen Gewebe. Hier ist vor allem der Herzwurm des Hundes, Dirofilaria immitis, zu nennen. Die Verbindungen der Formel I sind gegen diese Parasiten hoch wirksam.

Weiter sind die Verbindungen der Formel I zur Bekämpfung von humanpathogenen Parasiten geeignet, unter denen als typische, im Verdauungstrakt vorkommende Vertreter solche der Arten Ancylostoma, Necator, Ascaris, Strongyloides, Trichinella, Capillaria, Trichuris und Enterobius zu nennen sind. Wirksam sind die Verbindungen vorliegender Erfindung auch gegen Parasiten der Arten Wuchereria, Brugia, Onchocerca und Loa aus der Familie der Filariidae, die im

Blut, im Gewebe und verschiedenen Organen vorkommen, ferner gegen Dracunculus und Parasiten der Arten Strongyloides und Trichinella, die speziell den Gastro-Intestinalkanal infizieren.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Verbindungen der Formel I werden bei Warmblütern in Aufwandmengen von 0,01 bis 10 mg/kg Körpergewicht dosiert. Ueber geschlossenen Kultur-Anbauflächen werden sie in Mengen von 10 g bis 1000 g pro Hektar angewendet. Sie kommen ferner in Pferchen, Gehegen, Stallungen oder sonstigen Räumen zur Anwendung.

Die Formulierungen, d.h. die den Wirkstoff der Formel I enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxyd-Adduktes oder Phospholipide in Frage.

Die in der Formulierungstechnik gebräuchlichen Tenside sind u.a. in folgender Publikation beschrieben:

"1985 International McCutcheon's Emulsifiers and Detergents"
The Manufacturing Confectioner Publishing Co.,
Glen Rock New Jersey, USA.

Die pestiziden Zubereitungen enthalten in der Regel 0,01 bis 95 %, insbesondere 0,1 bis 80 %, Wirkstoff der Formel I, 5 bis 99,99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel mit 1-10'000 ppm Wirkstoffgehalt.

Ein weiterer Gegenstand vorliegender Erfindung betrifft daher Schädlingsbekämpfungsmittel, die neben üblichen Trägerstoffen und/oder Verteilungsmitteln als Wirkstoff mindestens eine Verbindung der Formel I enthalten.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## Herstellungsbeispiele

### 1. Herstellung von Ausgangs- und Zwischenprodukten

**Beispiel A.1: Herstellung von 14,15-Epoxy-Milbemycin D (Formel VII)**

Zu einer Lösung von 550 mg Milbemycin D in 5 ml Dichlormethan wird unter Eiskühlung eine Lösung von 170 mg Chlorperbenzoesäure in 5 ml Dichlormethan gegeben. Nach 1-stündigem Rühren bei 0° bis +5°C werden nochmals 170 mg des Oxidationsmittels hinzugefügt und weitere 30 min gerührt. Nach beendeter Reaktion wird die Lösung in eine eisgekühlte Lösung von Natriumsulfit gegossen und mit Essigsäureethylester extrahiert. Die vereinigten Extrakte werden einmal mit Wasser gewaschen, getrocknet und im Vakuum eingedampft. Das Rohprodukt wird durch Chromatographierung über eine Silicagel-Säule (Elutionsmittel n-Hexan/Essigsäureethylester 20:15) gereinigt. Es werden 450 mg 14,15-Epoxy-Milbemycin D als amorphe, weisse Substanz erhalten.

**Beispiel A.2: Herstellung von 15-Hydroxy-$\Delta^{13,14}$-Milbemycin D (Formel VI)**

Zu einer Lösung von 2,1 ml (1,75 g, 15,3 mmol) Triethylaluminium in 8,5 ml abs. Diethylether werden bei -20°C 9,5 ml (0,41 g, 9,53 mmol) einer 6,96 %igen Lösung von $HN_3$ in Diethylether gegeben. Diese Mischung wird dann bei -10°C unter stark exothermer Reaktion zu 1,8 g (3,15 mmol) 14,15-Epoxy-Milbemycin D (in Substanz) gegeben.

Nach 1 Stunde bei Raumtemperatur werden 4 ml absoluter Ether zugegeben und das gallertartige Reaktionsgemisch wird kräftig gerührt. Nach 4 Stunden wird wie im Beispiel A.1 aufgearbeitet. Chromatographische Reinigung an 70 g Kieselgel ($CH_2Cl_2$/Aceton 10:1) ergibt 200 mg (10 %) 14-Azido-15-hydroxy-Milbemycin D und 820 mg (45 %) 15-Hydroxy-$\Delta^{13,14}$-Milbemycin D, Smp.: 151-153°C (aus Methanol).

Beispiel A.3: Herstellung von 5-O-t-Butyldimethylsilyl-14,15-epoxy-Milbemycin D (Formel VII)

Eine Lösung von 2,21 g (3,86 mmol) 14,15-Epoxy-Milbemycin D, 757 mg (5,02 mmol) t-Butyl-dimethylchlorsilan und 342 mg (5,02 mmol) Imidazol in 4 ml Dimethylformamid wird 90 min bei Raumtemperatur gerührt. Anschliessend werden 80 ml Diethylether zugegeben, und das Gemisch wird über 20 g Kieselgel filtriert und eingeengt. Es werden 2,65 g (100 %) 5-O-t-Butyldimethylsilyl-14,15-epoxy-Milbemycin D erhalten.

$^1$H-NMR (300 MHz. Lösungsmittel $CDCl_3$. Messwerte $\delta$ bezogen auf $Si(CH_3)_4$ (= Tetramethylsilan = TMS)).

0,12 ppm (s) $(CH_3)_2Si$-O-;

0,92 ppm (s) (t.-$C_4H_9$)Si-O-;

1,23 ppm (breites s) $C_{14}CH_3$, d.h. Signal der $CH_3$-Gruppe in 14-Position);

2,56 ppm (d; J = 9 Hz) ($C_{15}H$, d.h. Signal des Protons in 15-Position).

Aehnlich lässt sich durch Reaktion mit Trimethylsilyl-trifluor-methansulfonat das entsprechende 5-O-Trimethylsilyl-14,15-epoxy-Milbemycin D herstellen, Smp. 92-97°C.

Beispiel A.4: Herstellung von 5-O-t-Butyldimethylsilyl-15-hydroxy-$\Delta^{13,14}$-Milbemycin D (Formel VI)

Eine Lösung des $HN_3$/$Et_3Al$-Komplex-Reagenz (hergestellt aus einer Lösung von 4,97 ml Triethylaluminium in 7 ml abs. Tetrahydrofuran (THF) und 9,15 ml einer 2,39 M Lösung von $HN_3$ (21,9 mmol) in abs. Diethylether) wird unter Argon zu einer Lösung von 5,0 g (7,29 mmol) 5-O-t-Butyldimethylsilyl-14,15-epoxy-Milbemycin D in ca. 20 ml abs. Tetrahydrofuran (THF) gegeben, und das Gemisch wird 15 Stunden unter Rückfluss erhitzt. Anschliessend werden bei Raumtemperatur 250 ml Ether, 2 ml Methanol und schliesslich ein Gemisch von 10 g $Na_2SO_4\cdot 10\ H_2O$ und 10 g Celite zugegeben. Das Gemisch wird filtriert und eingeengt. Die chromatographische Reinigung des Rohproduktes an 160 g Kieselgel (0 bis 30 % Essigsäureethylester in Hexan) ergibt 2,37 g (47 %) 5-O-t-Butyldimethylsilyl-15-hydroxy-$\Delta^{13,14}$-Milbemycin D.

$^1$H-NMR (300 MHz, $CDCl_3$):

1,59 ppm(d; J = 1 Hz), ($C_{14}CH_3$);

4,06 ppm (dd; $J_1$ = 11 Hz; $J_2$ = 4 Hz) ($C_{15}H$);

5,15 ppm (d; J = 8 Hz) ($C_{13}H$):

Daneben werden 109 mg (2 %) 13ß-Azido-5-O-t-butyldimethylsilyl-Milbemycin D gewonnen.

Beispiel A.5: Herstellung von 14,15-Epoxy-Milbemycin $A_4$ ($R_2$=$C_2H_5$) [Formel VII]

Zu einer Lösung von 5,7 g (10,5 mmol) Milbemycin $A_4$ in 140 ml Dichlormethan und 120 ml 0,5 M $NaHCO_3$-Lösung wird bei Raumtemperatur eine Lösung von 2,43 g (14,08 mmol) m-Chlorobenzopersäure in 70 ml Dichlormethan tropfenweise zugegeben. Das Gemisch wird 1 Stunde bei Raumtemperatur kräftig gerührt und dann mit 300 ml Dichlormethan verdünnt. Die organische Phase wird mit wässriger $NaHCO_3$-Lösung gewaschen, mit $Na_2SO_4$ getrocknet und eingeengt. Es werden 5,7 g Epoxyd als Rohprodukt erhalten.

Beispiel A.6: Herstellung von 5-O-t-Butyldimethylsilyl-14,15-epoxy-Milbemycin $A_4$ (Formel VII)

5,7 g 14,15-Epoxy-Milbemycin $A_4$ werden in 10 ml trockenem Dimethylformamid (DMF) gelöst. Bei Raumtemperatur werden 0,63 g (9,16 mmol) Imidazol und 1,4 g (9,34 mmol) t-Butyldimethylchlorsilan zugegeben. Das Gemisch wird 1 Stunde bei Raumtemperatur gerührt und an 150 g Kieselgel chromatographiert (Hexan/Ether 4:1), wobei 2,84 g (40 % d.Th. Ausbeute, bezogen auf Milbemycin $A_4$) des silylierten Epoxi-Derivats erhalten werden.

Beispiel A.7: Herstellung von 5-O-t-Butyldimethylsilyl-15-hydroxy-$\Delta^{13,14}$-Milbemycin $A_4$ (Formel VI)

Das Komplex-Reagenz $HN_3$/Al(Ethyl)$_3$ wird wie folgt hergestellt: 2,8 ml (12,2 mmol) $Al(C_2H_5)_3$ in 4 ml abs.Tetrahydrofuran (THF) werden unter Argon bei ca. -20°C langsam mit 5,28 ml (20,4 mmol) einer 10 %igen Lösung von $HN_3$ in abs. Diethylether versetzt. Zu dieser Lösung wird unter Argon eine Lösung von 2,84 g (4,25 mmol) 5-O-t-Butyldimethylsilyl-14,15-epoxy-milbemycin $A_4$ (Bsp. A.6) in ca. 12 ml abs. Tetrahydrofuran gegeben, und das so erhaltene Gemisch wird 4 Stunden unter Rückfluss erhitzt. Bei Raumtemperatur werden 500 ml Diethylether, 10 g $Na_2SO_4 \cdot 10H_2O$ und 10 g Celite zugegeben, und das Gemisch wird filtriert und eingeengt. Die Chromatographie des Rohproduktes an 100 g Kieselgel (Hexan/ Diethylether 7:2) ergibt 1,72 g (60 % d.Th.) der Titel-Verbindung.
$^1$H-NMR (300 MHz, $CDCl_3$):
1,59 ppm (br. s) ($C_{14}CH_3$);
4,05 ppm (br. s) ($C_{15}H$);
5,15 ppm (d; J = 6 Hz) ($C_{13}H$).

Daneben werden 0,1 g 13ß-Azido-5-O-t-butyldimethylsilyl-Milbemycin $A_4$ erhalten.

Beispiel A.8: Herstellung von 15-Hydroxy-$\Delta^{13,14}$-Milbemycin $A_4$ (Formel VI)

Die Hydrolyse von 5-O-t-Butyldimethylsilyl-15-hydroxy-$\Delta^{13,14}$-Milbemycin $A_4$ (Beispiel A.7) mit einer 1 %igen Lösung von p-Toluolsulfonsäure in Methanol und die Aufarbeitung in Diethylether mit 5%iger wässriger Na-Hydrogencarbonat-Lösung ergibt die Titel-Verbindung.

Beispiel A.9: Herstellung von 14,15-Epoxy-Milbemycin $A_3$ ($R_2$=$CH_3$) (Formel VII)

Nach der Vorschrift des Beispiels A.1 werden aus 220 mg Milbemycin $A_3$ in 5 ml Dichlormethan und 320 mg Benzoepersäure in 5 ml Dichlormethan bei -2° bis +5°C während 1,5 Stunden und Reinigung über eine Silicagel-Säule 190 mg 14,15-Epoxy-Milbemycin $A_3$ gewonnen.

Beispiel A.10: Herstellung von 5-O-tert.-Butyldimethylsilyl-14,15-epoxy-Milbemycin $A_3$ (Formel VII)

Nach der Vorschrift des Beispiels A.3 werden aus 190 mg 14,15-Epoxi-Milbemycin $A_3$ und 120 mg tert.-Butyldimethylchlorsilan in Gegenwart von Imidazol 217 mg der Titel-Verbindung erhalten.

Beispiel A.11: Herstellung von 5-O-tert.-Butyldimethylsilyl-15-hydroxy-$\Delta^{13,14}$-Milbemycin $A_3$ (Formel VI)

Analog zur Epoxyd-Spaltung des Beispiels A.4 werden aus 210 mg 5-O-tert.-Butyldimethylsilyl-14,15-epoxy-Milbemycin $A_3$ in absolutem Diethylether mit Hilfe des Komplex-Reagenz $HN_3/Et_3Al$ unter Argon nach anschliessender Reinigung 203 mg der Titelverbindung erhalten.
$^1$H-NMR (300 HMz, $CDCl_3$):
1,58 ppm (br. s) ($C_{14}CH_3$);
4,05 ppm (br. s) ($C_{15}H$);
5,15 ppm (d; J=6 Hz) ($C_{13}H$).

Beispiel A.12: Herstellung von 15-Hydroxy-$\Delta^{13,14}$-Milbemycin $A_3$ (Formel VI)

Analog zu Beispiel A.4 wird das Reagenz $HN_3/Al(C_2H_5)_3$ frisch hergestellt und bei -10°C zu einer Lösung von 830 mg (3,05 mmol) 14,15-Epoxy-Milbemycin $A_3$ in 7 ml trockenem Diethylether getropft. Nach der Aufarbeitung werden 385 mg 15-Hydroxy-$\Delta^{13,14}$-Milbemycin $A_3$ und 92 mg 14-Azido-15-hydroxy-Milbemycin $A_3$ erhalten.

Beispiel A.13: Herstellung von 13-Deoxy-14,15-epoxy-22,23-dihydro-avermectin-Bla-aglykon ($R_2$ = sec.$C_4H_9$) (Formel VII)

Analog zu Beispiel A.1 erhält man aus 520 mg 13-Deoxi-22,23-dihydro-avermectin-Bla-aglykon [US-PS 4,173,571 und Tetrahedron Letters, Vol. 24, No. 48, pp. 5333-5336 (1983)] und 210 mg m-Chlorbenzoepersäure in 20 ml Dichlormethan 510 mg der Titelverbindung.

Beispiel A.14: Herstellung von 5-O-t.Butyldimethylsilyl-13-deoxy-14,15-epoxy-22,23-dihydro-avermectin-Bla-aglykon (Formel VII)

Analog zu Beispiel A.3 erhält man aus 220 mg 13-Deoxy-14,15-epoxy-22,23-dihydro-avermectin-Bla-aglykon (Beispiel A.13) und 55 mg tert.Butyldimethylchlorsilan in Gegenwart von 25 mg Imidazol in 5 ml trockenem DMF 108 mg der Titelverbindung.

Beispiel A.15: Herstellung von 13-Deoxy-15-hydroxy$\Delta^{13,14}$-22,23-dihydro-avermectin-Bla-aglykon (Formel VI)

Analog zu Beispiel A.4 erhält man aus 220 mg 13-Deoxy-14,15-epoxi-22,23-dihydro-avermectin-Bla-aglykon (Beispiel A.13) mit dem Komplex-Reagenz, bestehend aus 320 mg $Al(C_2H_5)_3$ und 110 mg einer 6,96 %igen Lösung von $HN_3$ in total 16 ml trockenem Diethylether 112 mg der Titelverbindung. Daneben werden 61 mg 13-Deoxy-14-azido-15-hydroxy-22,23-dihydro-avermectin-Bla-aglykon erhalten.

**Beispiel A.16:** Herstellung von 5-O-tert.Butyldimethylsilyl-13ß-hydroxy-milbemycin D und von 13ß-Hydroxy-milbemycin D (Formel II)

Eine Lösung bestehend aus 286 mg (0,41 mmol) 5-O-tert.Butyldimethylsilyl-15-hydroxy-$\Delta^{13,14}$-milbemycin D und 209 mg (0,56 mmol) Pyridiniumdichromat (PDC) in 3 ml Dimethylformamid (DMF) wird 30 min bei Raumtemperatur gerührt. Anschliessend wird 1 ml Isopropanol zugegeben, 5 min weitergerührt und dann mit 50 ml Ether verdünnt. Nach weiteren 10 min wird das Gemisch durch Kieselgel filtriert und eingeengt. Nach chromatographischer Reinigung des Rohproduktes an 20 g Kieselgel (Ether/Hexan 1:2) werden 165 mg (57 %) 5-O-t-Butyldimethylsilyl-13ß-hydroxy-Milbemycin D erhalten.
$^1$H-NMR (300 MHz; $CDCl_3$; TMS):
1,59 ppm (br. s)($C_{14}CH_3$)
3,70 ppm (d; J = 10 Hz)($C_{13}H$).

105 mg (0,153 mmol) der so gewonnenen Verbindung werden mit 1 ml einer 1 %igen Lösung von p-Toluolsulfonsäure in Methanol 1 Stunde bei Raumtemperatur gerührt. Das Gemisch wird mit 20 ml Ether verdünnt, durch Kieselgel filtriert und eingeengt. Der Rückstand wird an ca. 10 g Kieselgel chromatographiert (Aceton/Dichlormethan 1:4), wobei 73 mg (83 %) 13ß-Hydroxy-Milbemycin D erhalten werden.
$^1$H-NMR (300 MHz; $CDCl_3$; TMS):
1,58 ppm (br.s)($C_{14}CH_3$)
3,71 ppm (d; J = 10 Hz)($C_{13}H$).

**Beispiel A.17:** Herstellung von 5-O-tert.Butyldimethylsilyl-13ß-hydroxy-milbemycin $A_4$

Eine Lösung bestehend aus 1,06 g (1,59 mmol) 5-O-tert.Butyldimethylsilyl-15-hydroxy-$\Delta^{13,14}$-milbemycin $A_4$ und 383 mg (1,02 mmol) Pyridiniumdichromat (PDC) in 5 ml Dimethylformamid (DMF) wird 30 min. bei Raumtemperatur gerührt. Anschliessend wird 1 ml Isopropanol zugegeben, 5 min. weitergerührt und dann mit 50 ml Ether verdünnt. Nach weiteren 10 min. wird das Gemisch durch Kieselgel

filtriert und eingeengt. Nach chromatographischer Reinigung des Rohproduktes an 20 g Kieselgel (Ether/Hexan 1:2) werden 625 mg (59 %) 5-O-t-Butyldimethylsilyl-13β-hydroxy-Milbemycin $A_4$ erhalten.

$^1$H-NMR (300 MHz; $CDCl_3$; TMS):

0,98 ppm (t; J = 7 Hz) ($\underline{CH_3}CH_2$)

1,95 ppm (br. s) ($C_{14}CH_3$)

3,69 ppm (d; J = 9 Hz) ($C_{13}H$)

2. Herstellung der Endprodukte

Beispiel E.1: Herstellung von 5-O-tert.-Butyldimethylsilyl-13β-(methoxycarbonyloxy)-milbemycin $A_4$

Zu einer Lösung von 44 mg 5-O-tert.-Butyldimethylsilyl-13β-hydroxy-milbemycin $A_4$ und 32 µl Pyridin in 2 ml Methylenchlorid werden 16 µl Chlorameisensäuremethylester gegeben. Anschliessend wird das Reaktionsgemisch 3 Stunden bei Raumtemperatur gerührt. Die Aufarbeitung und chromatographische Reinigung an Kieselgel (Hexan/Diethylether 2:1) ergibt 44 mg Produkt.

$^1$H-NMR (300 MHz; $CDCl_3$):

3,03 ppm (dt; $J_d$ = 3 Hz, $J_t$ = 9 Hz) ($C_{25}H$)

3,73 ppm (s) ($CH_3O$)

4,72 ppm (d, J = 11 Hz) ($C_{13}H$)

Beispiel E.2: Herstellung von 13β-(Methoxycarbonyloxy)-milbemycin $A_4$

Eine Lösung von 42 mg 5-O-tert.-Butyldimethylsilyl-13β-methoxy-carbonyloxy-milbemycin $A_4$ in 2,0 ml 40 % HF/$CH_3CN$ 5:95 wird 1 Std. bei Raumtemperatur gerührt. Aufarbeitung und Chromatographie an Kieselgel mit Hexan/Essigsäureethylester (2:1) ergeben 30 mg Produkt.

$^1$H-NMR (300 MHz, $CDCl_3$):

3,02 ppm (dt, $J_d$ = 3 Hz, $J_t$ = 9 Hz) ($C_{25}H$)

3,75 ppm (s) ($CH_3O$)

4,73 ppm (d, J = 10 Hz) ($C_{13}H$)

Beispiel E.3: Herstellung von 5-O-tert.-Butyldimethylsilyl-13β-(cyclohexyloxycarbonyloxy)-milbemycin $A_4$

Zu einer Lösung von 100 mg 5-O-tert.-Butyldimethylsilyl-13β-hydroxy-milbemycin $A_4$ und 72 µl Pyridin in 2 ml Methylenchlorid werden 66 µl Chlorameisensäurecyclohexylester gegeben. Das Reaktionsgemisch wird 5 Stunden bei Raumtemperatur gerührt. Aufarbeitung und Chromatographie an Kieselgel mit Hexan/Essigsäureethylester ergeben 101 mg Produkt.

$^1$H-NMR (300 MHz, $CDCl_3$):

3,02 ppm (dt, $J_d$ = 3 Hz, $J_t$ = 9 Hz) ($C_{25}H$)

4,54 ppm (m) ($CH_2C\underline{H}(O)CH_2$)

4,73 ppm (d, J = 12 Hz) ($C_{13}H$)

Beispiel E.4: Herstellung von 13β-(Cyclohexyloxycarbonyloxy)-milbemycin $A_4$

Eine Lösung von 95 mg 5-tert.-Butyldimethylsilyl-13β-cyclohexyloxy-carbonyloxy-milbemycin $A_4$ in 1 ml 40 % $HF/CH_3CN$ 5:95 wird 1 Stunde bei Raumtemperatur gerührt. Aufarbeitung und Chromatographie an Kieselgel mit Hexan/Essigsäureethylester 2:1 ergeben 68 mg Produkt.

$^1$H-NMR (300 MHz, $CDCl_3$):

3,02 ppm (dt, $J_d$ = 3 Hz, $J_t$ = 9 Hz) ($C_{25}H$)

4,56 ppm (m, symmetrisches 7-Linien-System) ($CH_2\underline{CH}(O)CH_2$)

4,73 ppm (d, J = 12 Hz) ($C_{13}H$)

Nach den vorstehend beschriebenen Verfahren lassen sich beispielsweise auch folgende Verbindungen der Formel I herstellen:

**Beispiel E.5:** 5-O-tert.-Butyldimethylsilyl-13β-(methoxycarbonyloxy)-milbemycin D

Massenspektrum (MS) (m/e): 744 ($M^+$)
$^1$H-NMR (250 MHz, $CDCl_3$):
3,04 ppm (bd, J = 9 Hz) ($C_{25}H$)
3,76 ppm (s) ($CH_3O$)
4,73 ppm (d, J = 11 Hz) ($C_{13}H$)

**Beispiel E.6:** 13β-(Methoxycarbonyloxy)-milbemycin D
Massenspektrum (MS) (m/e): 630 ($M^+$)
$^1$H-NMR (250 MHz, $CDCl_3$):
3,05 ppm (bd, J = 9 Hz) ($C_{25}H$)
3,78 ppm (s) ($CH_3O$)
4,74 ppm (d, J = 11 Hz) ($C_{13}H$)

**Beispiel E.7:** 5-O-tert.-Butyldimethylsilyl-13β-(vinyloxycarbonyloxy)-milbemycin D

Massenspektrum (MS) (m/e): 756 ($M^+$)
$^1$H-NMR (250 MHz, $CDCl_3$):
3,03 ppm (bd, J = 9 Hz) ($C_{25}H$)
4,57 ppm (dd, $J_1$= 7 Hz, $J_2$ = 2 Hz) ($\underline{H_c}H_tC{=}CHO$)
4,77 ppm (d, J = 11 Hz) ($C_{13}H$)
4,95 ppm (dd, $J_1$= 15 Hz, $J_2$ = 2 Hz) ($H_c\underline{H_t}C{=}CHO$)
7,07 ppm (dd, $J_1$= 15 Hz, $J_2$ = 7 Hz) ($H_cH_tC{=}C\underline{H}O$)

**Beispiel E.8:** 13β-(Vinyloxycarbonyloxy)-milbemycin D
$^1$H-NMR (250 MHz, $CDCl_3$):
3,04 ppm (bd, J = 9 Hz) ($C_{25}H$)
4,57 ppm (dd, $J_1$= 7 Hz, $J_2$ = 2 Hz) ($\underline{H_c}H_tC{=}CHO$)
4,78 ppm (d, J = 11 Hz) ($C_{13}H$)
4,92 ppm (dd, $J_1$= 15 Hz, $J_2$ = 2 Hz) ($H_c\underline{H_t}C{=}CHO$)
7,08 ppm (dd, $J_1$= 15 Hz, $J_2$ = 7 Hz) ($H_cH_tC{=}C\underline{H}O$)

Beispiel E.9: 5-tert.-Butyldimethylsilyl-13β-(allyloxycarbonyloxy)-milbemycin D

Massenspektrum (MS) (m/e): 770 ($M^+$)

$^1H$-NMR (250 MHz, $CDCl_3$):

3,04 ppm (bd, J = 9 Hz) ($C_{25}H$)

4,60 ppm (m) (C(13)-O$C\underline{H}_2$)

4,74 ppm (d, J = 11 Hz) ($C_{13}H$)

Beispiel E.10: 13β-(Allyloxycarbonyloxy)-milbemycin D

$^1H$-NMR (250 MHz, $CDCl_3$):

3,04 ppm (bd, J = 9 Hz) ($C_{25}H$)

3,61 ppm (m) (C(13)-O$C\underline{H}_2$)

3,74 ppm (d, J = 11 Hz) ($C_{13}H$)

Beispiel E.11: 13β-(Ethoxycarbonyloxy)-milbemycin $A_4$

Massenspektrum (MS) (m/e): 630 ($M^+$)

$^1H$-NMR (270 MHz, $CDCl_3$):

3,96 ppm (d, J = 6 Hz) ($C_6H$)

4,74 ppm (d, J = 11 Hz) ($C_{13}H$)

Beispiel E.12: 13β-[(2,2,2-Trichlorethoxy)-carbonyloxy]-milbemycin $A_4$

Massenspektrum (MS) (m/e): 732 ($M^+$)

$^1H$-NMR (270 MHz, $CDCl_3$):

3,97 ppm (d, J = 6 Hz) ($C_6H$)

4,68 ppm (d, J = 9 Hz) ($C_{13}H$)

Beispiel E.13: 13β-[(2,2-Dimethyl-1,3-dioxolan-4-yl)-methoxy-carbonyloxy]-milbemycin $A_4$

Massenspektrum (MS) (m/e): 716 ($M^+$)

$^1$H-NMR (270 MHz, $CDCl_3$):

1,42 ppm (s) ( 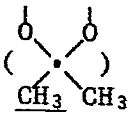 )

1,44 ppm (s) ( 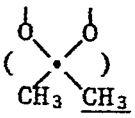 )

3,97 ppm (d, J = 6 Hz) ($C_6H$)

Beispiel E.14: 13β-[(2,3-Dihydroxypropoxy)-carbonyloxy]-milbemycin $A_4$

$^1$H-NMR (270 MHz, $CDCl_3$):

3,04 ppm (dt, $J_d$ = 3 Hz, $J_t$ = 9 Hz) ($C_{25}H$)

3,97 ppm (d, J = 6 Hz ($C_6H$)

4,75 ppm (d, J = 10 Hz) ($C_{13}H$)

Beispiel E.15: 5-O-tert.-Butyldimethylsilyl-13β-(2,2,2-trichlor-tert.-butoxy)-carbonyloxy-milbemycin $A_4$

Massenspektrum (MS) (m/e): 878, 876 ($M^+$,$C_{43}H_{65}Cl_3O_{10}Si$), 874

$^1$H-NMR (300 MHz, $CDCl_3$):

1,89 ppm (s) ($CCl_3C(\underline{CH_3})(CH_3)O$)

1,90 ppm (s) ($CCl_3C(CH_3)(\underline{CH_3})O$)

3,03 ppm (dt, $J_d$ = 2,5 Hz, $J_t$ = 9 Hz) ($C_{25}H$)

4,71 ppm (d, J = 11 Hz) ($C_{13}H$)

Beispiel E.16: 13β-(2,2,2-Trichlor-tert.-butoxy)-carbonyloxy-milbemycin $A_4$

Massenspektrum (MS) (m/e): 764, 762 ($M^+$,$C_{37}H_{57}Cl_3O_{10}$), 760
$^1$H-NMR (300 MHz, $CDCl_3$):
1,89 ppm (s) ($CCl_3C(\underline{CH_3})(CH_3)O$)
1,90 ppm (s) ($CCl_3C(CH_3)(\underline{CH_3})O$)
3,03 ppm (dt, $J_d$ = 2,5 Hz, $J_t$ = 9 Hz) ($C_{25}H$)
4,28 ppm (t, J = 7 Hz) ($C_5H$)
4,71 ppm (d, J = 11 Hz) ($C_{13}H$)

Beispiel E.17: 5-O-tert.-Butyldimethylsilyl-13β-isopropyloxycarbonyloxy-milbemycin $A_4$

Massenspektrum (MS) (m/e): 758 ($M^+$,$C_{42}H_{66}O_{10}Si$)
$^1$H-NMR (300 MHz, $CDCl_3$):
3,03 ppm (dt, $J_d$ = 2,5 Hz, $J_t$ = 9 Hz) ($C_{25}H$)
4,42 ppm (bs, $W_{\frac{1}{2}}$ = 11) ($C_5H$)
4,73 ppm (d, J = 11 Hz) ($C_{13}H$)
4,84 ppm (symmetrisches 7-dinien-System) ($(CH_3)_2C\underline{H}O$)

Beispiel E.18: 13β-Isopropyloxycarbonyloxy-milbemycin $A_4$
Massenspektrum (MS) (m/e): 644 ($M^+$,$C_{36}H_{52}O_{10}$)
$^1$H-NMR (300 MHz, $CDCl_3$):
3,03 ppm (dt, $J_d$ = 2,5 Hz, $J_t$ = 9 Hz) ($C_{25}H$)
4,28 ppm (t, J = 7 Hz) ($C_5H$)
4,72 ppm (d, J = 11 Hz) ($C_{13}H$)
4,83 ppm (symmetrisches 7-dinien-System) ($(CH_3)_2C\underline{H}O$)

Beispiel E.19: 5-O-tert.-Butyldimethylsilyl-13β-phenoxycarbonyloxy-milbemycin $A_4$

Massenspektrum (MS) (m/e): 792 ($M^+$,$C_{45}H_{64}O_{10}Si$)
$^1H$-NMR (300 MHz, $CDCl_3$):
3,03 ppm (dt, $J_d$ = 2,5 Hz, $J_t$ = 9 Hz) ($C_{25}H$)
4,42 ppm (bs, $W_{\frac{1}{2}}$ = 11) ($C_5H$)
4,81 ppm (d, J = 11 Hz) ($C_{13}H$)
7,1 - 7,45 ppm (m) ($C_6H_5$)

Beispiel E.20: 13β-Phenoxycarbonyloxy-milbemycin $A_4$
Massenspektrum (MS) (m/e): 678 ($M^+$,$C_{39}H_{50}O_{10}$)
$^1H$-NMR (300 MHz, $CDCl_3$):
3,03 ppm (dt, $J_d$ = 2,5 Hz, $J_t$ = 9 Hz) ($C_{25}H$)
4,29 ppm (t, J = 7 Hz) ($C_5H$)
4,82 ppm (d, J = 11 Hz) ($C_{13}H$)
7,1 - 7,4 ppm (m) ($C_6H_5$)

Beispiel E.21: 5-O-tert.-Butyldimethylsilyl-13β-(4-nitrophenoxy)-carbonyl-milbemycin $A_4$

Massenspektrum (MS) (m/e): 838 ($M^+$+1,$C_{45}H_{63}NO_{12}Si$)
$^1H$-NMR (300 MHz, $CDCl_3$):
3,03 ppm (dt, $J_d$ = 2,5 Hz, $J_t$ = 9 Hz) ($C_{25}H$)
4,42 ppm (bs, $W_{\frac{1}{2}}$ = 11) ($C_5H$)
4,83 ppm (d, J = 11 Hz) ($C_{13}H$)
7,38 ppm (m) ($C_6\underline{H_2}H_2$-O)
8,27 ppm (m) ($C_6H_2\underline{H_2}$-O)

Beispiel E.22: 13β-(4-Nitrophenoxy)-carbonyloxy-milbemycin $A_4$

Massenspektrum (MS) (m/e): 723 ($M^+$,$C_{39}H_{49}NO_{12}$)

$^1$H-NMR (300 MHz, $CDCl_3$):

3,03 ppm (dt, $J_d$ = 2,5 Hz, $J_t$ = 9 Hz) ($C_{25}H$)

4,29 ppm (bd), J = 5 Hz) ($C_5H$)

4,85 ppm (d, J = 10 Hz) ($C_{13}H$)

7,37 ppm (m) ($C_6\underline{H}_2H_2$-O)

8,27 ppm (m) ($C_6H_2\underline{H}_2$-O)

Beispiel E.23: 5-O-tert.-Butyldimethylsilyl-13β-(4-methoxy-carbonylphenoxy)-carbonyloxy-milbemycin $A_4$

Massenspektrum (MS) (m/e): 850 ($M^+$,$C_{47}H_{66}O_{12}Si$)

$^1$H-NMR (300 MHz, $CDCl_3$):

3,03 ppm (dt, $J_d$ = 2,5 Hz, $J_t$ = 9 Hz) ($C_{25}H$)

3,88 ppm (s) ($CH_3O$)

4,42 ppm (bs, $W_{\frac{1}{2}}$ = 10) ($C_5H$)

4,92 ppm (d, J = 11 Hz) ($C_{13}H$)

7,25 ppm (m) ($C_6\underline{H}_2H_2$-O)

8,06 ppm (m) ($C_6H_2\underline{H}_2$-O)

Beispiel E.24: 13β-(4-Methoxycarbonylphenoxy)-carbonyloxy-milbemycin $A_4$

Massenspektrum (MS) (m/e): 736 ($M^+$,$C_{41}H_{52}O_{12}$)

$^1$H-NMR (300 MHz, $CDCl_3$):

3,03 ppm (dt, $J_d$ = 2,5 Hz, $J_t$ = 9 Hz) ($C_{25}H$)

3,88 ppm (s) ($CH_3O$)

4,29 ppm (bs, $W_{\frac{1}{2}}$ = 14) ($C_5H$)

4,83 ppm (d, J = 11 Hz) ($C_{13}H$)

7,26 ppm (m) ($C_6\underline{H}_2H_2$-O)

8,06 ppm (m) ($C_6H_2\underline{H}_2$-O)

Beispiel E.25: 5-O-tert.-Butyldimethylsilyl-13β-benzyloxycarbonyloxy-milbemycin $A_4$

Massenspektrum (MS) (m/e): 806 ($M^+$,$C_{46}H_{66}O_{10}Si$)
$^1$H-NMR (300 MHz, $CDCl_3$):
3,03 ppm (dt, $J_d$ = 2,5 Hz, $J_t$ = 9 Hz) ($C_{25}H$)
4,41 ppm (bs, $W_{\frac{1}{2}}$ = 11) ($C_5H$)
4,74 ppm (d, J = 11 Jz) ($C_{13}H$)
5,10 ppm (d, J = 13 Hz) ($C_6H_5C\underline{H}HO$)
5,16 ppm (d, J = 13 Hz) ($C_6H_5CH\underline{H}O$)
7,34 ppm (m) ($C_6H_5$)

Beispiel E.26: 13β-Benzyloxycarbonyloxy-milbemycin $A_4$
Massenspektrum (MS) (m/e): 692 ($M^+$,$C_{40}H_{52}O_{10}$)
$^1$H-NMR (250 MHz, $CDCl_3$):
3,03 ppm (bt, J = 9 Hz) ($C_{25}H$)
4,30 ppm (t, J = 7 Hz) ($C_5H$)
4,76 ppm (d, J = 11 Hz) ($C_{13}H$)
5,11 ppm (d, J = 12 Hz) ($C_6H_5C\underline{H}HO$)
5,18 ppm (d, J = 12 Hz) ($C_6H_5CH\underline{H}O$)
7,36 ppm (m) ($C_6H_5$)

Beispiel E.27: 5-O-tert.-Butyldimethylsilyl-13β-(9-fluorenyl-methoxy)-carbonyloxy-milbemycin $A_4$

Massenspektrum (MS) (m/e): 894 ($M^+$,$C_{53}H_{70}O_{10}Si$)
$^1$H-NMR (300 MHz, $CDCl_3$):
3,03 ppm (dt, $J_d$ = 2,5 Hz, $J_t$ = 9 Hz) ($C_{25}H$)
4,25 ppm (t, J = 7,5 Hz) ($C\underline{H}CHHOCO$)
4,33 - 4,47 ppm (m, 3H) ($CHC\underline{HH}OCO$, $C_5\underline{H}$)
4,70 ppm (d, J = 10 Hz) ($C_{13}H$)

Beispiel E.28: 13β-(9-Fluorenyl-methoxy)-carbonyloxy-milbemycin $A_4$

Massenspektrum (MS) (m/e): 780 ($M^+$,$C_{47}H_{56}O_{10}$)

$^1$H-NMR (250 MHz, $CDCl_3$):

3,04 ppm (bt, J = 9 Hz) ($C_{25}H$)

3,21 - 3,35 ppm (m, 2H) (C<u>H</u>CHHOCO, $C_5H$)

3,35 - 3,51 ppm (m, 2H) (CHC<u>HH</u>OCO)

3,72 ppm (d, J = 10 Hz) ($C_{13}H$)

Beispiel E.29: 5-O-tert.-Butyldimethylsilyl-13β-(2-bromäthoxy)-carbonyloxy-milbemycin $A_4$

Massenspektrum (MS) (m/e): 824, 822 ($M^+$,$C_{41}H_{63}BrO_{10}Si$)

$^1$H-NMR (300 MHz, $CDCl_3$):

3,03 ppm (dt, $J_d$ = 2,5 Hz, $J_t$ = 9 Hz) ($C_{25}H$)

3,52 ppm (t, J = 6,5 Hz) ($BrCH_2$)

4,42 ppm (m, 3H) ($BrCH_2C\underline{H}_2O$, $C_5\underline{H}$)

4,73 ppm (d, J = 11 Hz) ($C_{13}H$)

Beispiel E.30: 13β-(2-Bromäthoxy)-carbonyloxy-milbemycin $A_4$

Massenspektrum (MS) (m/e): 710, 708 ($M^+$,$C_{35}H_{49}BrO_{10}$)

$^1$H-NMR (250 MHz, $CDCl_3$):

3,05 ppm (dt, $J_d$ = 2,5 Hz, $J_t$ = 9 Hz) ($C_{25}H$)

3,54 ppm (t, J = 6,5 Hz) ($BrCH_2$)

4,30 ppm (t, J = 7 Hz) ($C_5H$)

4,43 ppm (m) ($BrCH_2C\underline{H}_2O$)

4,76 ppm (d, J = 10 Hz) ($C_{13}H$)

Beispiel E.31: 5-O-tert.-Butyldimethylsilyl-13β-([-]-menthyloxy)-carbonyloxy-milbemyxin $A_4$

Massenspektrum (MS) (m/e): 854 ($M^+$,$C_{49}H_{78}O_{10}Si$)

$^1$H-NMR (250 MHz, $CDCl_3$):

3,04 ppm (bt, J = 9 Hz) ($C_{25}H$)

4,44 ppm (bs, $W_{\frac{1}{2}}$ = 11) ($C_5H$)

4,52 ppm (m) (CHOCOO)

4,76 ppm (d, J = 11 Hz) ($C_{13}H$)

Beispiel E.32: 13β-([-]-Menthyloxy)-carbonyloxy-milbemycin $A_4$

Massenspektrum (MS) (m/e): 740 ($M^+$, $C_{43}H_{64}O_{10}$)

$^1$H-NMR (300 MHz, $CDCl_3$):

3,03 ppm (bt, J = 9 Hz) ($C_{25}H$)

4,29 ppm (t, J = 7 Hz) ($C_5H$)

4,51 ppm (dt, $J_d$ = 4 Hz, $J_t$ = 11 Hz) (CHOCOO)

4,75 ppm (d, J = 10 Hz) ($C_{13}H$)

Beispiel E.33: 5-O-tert.-Butyldimethylsilyl-13β-methoxycarbonyloxy-milbemycin $A_3$

Massenspektrum (MS) (m/e): 716 ($M^+$, $C_{39}H_{60}O_{10}Si$)

$^1$H-NMR (300 MHz, $CDCl_3$):

3,25 ppm (m) ($C_{25}H$)

3,74 ppm (s) ($CH_3O$)

4,42 ppm (bs, $W_{\frac{1}{2}}$ = 11) ($C_5H$)

4,74 ppm (d, J = 11 Hz) ($C_{13}H$)

Beispiel E.34: 13β-Methoxycarbonyloxy-milbemycin $A_3$

Massenspektrum (MS) (m/e): 602 ($M^+$, $C_{33}H_{46}O_{10}$)

$^1$H-NMR (300 MHz, $CDCl_3$):

3,24 ppm (m) ($C_{25}H$)

3,72 ppm (s) ($CH_3O$)

4,28 ppm (t, J = 7 Hz) ($C_5H$)

4,73 ppm (d, J = 11 Hz) ($C_{13}H$)

Beispiel E.35: 13β-(3,3,3-Trifluor-2,2-dichlor-propyloxy)-carbonyloxy-milbemycin $A_4$

Massenspektrum (MS) (m/e): 766 ($M^+$, $C_{36}H_{47}Cl_2F_3O_{10}$)

$^1$H-NMR (300 MHz, $CDCl_3$):

3,05 ppm (dt, $J_d$ = 2,5 Hz, $J_t$ = 9 Hz) ($C_{25}H$)

4,30 ppm (t, J = 7 Hz) ($C_5H$)

4,78 ppm (d, J = 11 Hz) ($C_{13}H$)

Analog zu den beschriebenen Arbeitsweisen werden auch die nachfolgend zusammen mit Verbindungen der vorgängigen Beispiele genannten Verbindungen der Formel I hergestellt:

Tabelle 1
Typische Vertreter von Verbindungen der Formel I, worin $R_1$ Wasserstoff ist.

| Verb. Nr. | $R_2$ | $R_3$ |
|---|---|---|
| 1.1 | $CH_3$ | $CH_3$ |
| 1.2 | $C_2H_5$ | $CH_3$ |
| 1.3 | $C_3H_7$-iso | $CH_3$ |
| 1.4 | $C_4H_9$-sek | $CH_3$ |
| 1.5 | $CH_3$ | $C_2H_5$ |
| 1.6 | $C_2H_5$ | $C_2H_5$ |
| 1.7 | $C_3H_7$-iso | $C_2H_5$ |
| 1.8 | $C_4H_9$-sek | $C_2H_5$ |
| 1.9 | $CH_3$ | $CH(CH_3)_2$ |
| 1.10 | $C_2H_5$ | $CH(CH_3)_2$ |
| 1.11 | $C_3H_7$-iso | $CH(CH_3)_2$ |
| 1.12 | $C_4H_9$-sek | $CH(CH_3)_2$ |
| 1.13 | $CH_3$ | $C_4H_9$-tert. |
| 1.14 | $C_2H_5$ | $C_4H_9$-tert. |
| 1.15 | $C_3H_7$-iso | $C_4H_9$-tert. |
| 1.16 | $C_4H_9$-sek | $C_4H_9$-tert. |
| 1.17 | $CH_3$ | $CH_2CH_2Br$ |
| 1.18 | $C_2H_5$ | $CH_2CH_2Br$ |
| 1.19 | $C_3H_7$-iso | $CH_2CH_2Br$ |
| 1.20 | $C_4H_9$-sek | $CH_2CH_2Br$ |
| 1.21 | $CH_3$ | $CH_2CH_2Cl$ |
| 1.22 | $C_2H_5$ | $CH_2CH_2Cl$ |
| 1.23 | $C_3H_7$-iso | $CH_2CH_2Cl$ |
| 1.24 | $C_4H_9$-sek | $CH_2CH_2Cl$ |
| 1.25 | $CH_3$ | $CH_2CCl_3$ |
| 1.26 | $C_2H_5$ | $CH_2CCl_3$ |
| 1.27 | $C_3H_7$-iso | $CH_2CCl_3$ |
| 1.28 | $C_4H_9$-sek | $CH_2CCl_3$ |
| 1.29 | $CH_3$ | $CCl_3$ |
| 1.30 | $C_2H_5$ | $CCl_3$ |
| 1.31 | $C_3H_7$-iso | $CCl_3$ |
| 1.32 | $C_4H_9$-sek | $CCl_3$ |
| 1.33 | $CH_3$ | $C(CH_3)_2CCl_3$ |
| 1.34 | $C_2H_5$ | $C(CH_3)_2CCl_3$ |
| 1.35 | $C_3H_7$-iso | $C(CH_3)_2CCl_3$ |
| 1.36 | $C_4H_9$-sek | $C(CH_3)_2CCl_3$ |
| 1.37 | $CH_3$ | $CH{=}CH_2$ |
| 1.38 | $C_2H_5$ | $CH{=}CH_2$ |
| 1.39 | $C_3H_7$-iso | $CH{=}CH_2$ |
| 1.40 | $C_4H_9$-sek | $CH{=}CH_2$ |
| 1.41 | $CH_3$ | $CH_2{-}CH{=}CH_2$ |
| 1.42 | $C_2H_5$ | $CH_2{-}CH{=}CH_2$ |
| 1.43 | $C_3H_7$-iso | $CH_2{-}CH{=}CH_2$ |
| 1.44 | $C_4H_9$-sek | $CH_2{-}CH{=}CH_2$ |
| 1.45 | $CH_3$ | Benzyl |
| 1.46 | $C_2H_5$ | Benzyl |

Tabelle 1 (Fortsetzung)

| Verb. Nr. | $R_2$ | $R_3$ |
|---|---|---|
| 1.47 | $C_3H_7$-iso | Benzyl |
| 1.48 | $C_4H_9$-sek | Benzyl |
| 1.49 | $CH_3$ | p-Nitrobenzyl |
| 1.50 | $C_2H_5$ | p-Nitrobenzyl |
| 1.51 | $C_3H_7$-iso | p-Nitrobenzyl |
| 1.52 | $C_4H_9$-sek | p-Nitrobenzyl |
| 1.53 | $CH_3$ | Phenyl |
| 1.54 | $C_2H_5$ | Phenyl |
| 1.55 | $C_3H_7$-iso | Phenyl |
| 1.56 | $C_4H_9$-sek | Phenyl |
| 1.57 | $CH_3$ | p-Nitrophenyl |
| 1.58 | $C_2H_5$ | p-Nitrophenyl |
| 1.59 | $C_3H_7$-iso | p-Nitrophenyl |
| 1.60 | $C_4H_9$-sek | p-Nitrophenyl |
| 1.61 | $CH_3$ | p-Methoxycarbonylphenyl |
| 1.62 | $C_2H_5$ | p-Methoxycarbonylphenyl |
| 1.63 | $C_3H_7$-iso | p-Methoxycarbonylphenyl |
| 1.64 | $C_4H_9$-sek | p-Methoxycarbonylphenyl |
| 1.65 | $CH_3$ | 2,2-Dimethyl-1,3-dioxolan-4-ylmethyl |
| 1.66 | $C_2H_5$ | 2,2-Dimethyl-1,3-dioxolan-4-ylmethyl |
| 1.67 | $C_3H_7$-iso | 2,2-Dimethyl-1,3-dioxolan-4-ylmethyl |
| 1.68 | $C_4H_9$-sek | 2,2-Dimethyl-1,3-dioxolan-4-ylmethyl |
| 1.69 | $CH_3$ | $CH_2CH(OH)CH_2OH$ |
| 1.70 | $C_2H_5$ | $CH_2CH(OH)CH_2OH$ |
| 1.71 | $C_3H_7$-iso | $CH_2CH(OH)CH_2OH$ |
| 1.72 | $C_4H_9$-sek | $CH_2CH(OH)CH_2OH$ |
| 1.73 | $CH_3$ | $CH_2CH_2OCH_3$ |
| 1.74 | $C_2H_5$ | $CH_2CH_2OCH_3$ |
| 1.75 | $C_3H_7$-iso | $CH_2CH_2OCH_3$ |
| 1.76 | $C_4H_9$-sek | $CH_2CH_2OCH_3$ |
| 1.77 | $CH_3$ | $CH_2CH(C_2H_5)CH_2CH_2CH_2CH_3$ |
| 1.78 | $C_2H_5$ | $CH_2CH(C_2H_5)CH_2CH_2CH_2CH_3$ |
| 1.79 | $C_3H_7$-iso | $CH_2CH(C_2H_5)CH_2CH_2CH_2CH_3$ |
| 1.80 | $C_4H_9$-sek | $CH_2CH(C_2H_5)CH_2CH_2CH_2CH_3$ |
| 1.81 | $CH_3$ | Cyclohexyl |
| 1.82 | $C_2H_5$ | Cyclohexyl |
| 1.83 | $C_3H_7$-iso | Cyclohexyl |
| 1.84 | $C_4H_9$-sek | Cyclohexyl |
| 1.85 | $CH_3$ | $CH_2CH_2CH_2CH_2CH_2CH_3$ |
| 1.86 | $C_2H_5$ | $CH_2CH_2CH_2CH_2CH_2CH_3$ |
| 1.87 | $C_3H_7$-iso | $CH_2CH_2CH_2CH_2CH_2CH_3$ |
| 1.88 | $C_4H_9$-sek | $CH_2CH_2CH_2CH_2CH_2CH_3$ |
| 1.89 | $CH_3$ | 2-(1-Methylethyl)-5-methylcyclohexyl |
| 1.90 | $C_2H_5$ | 2-(1-Methylethyl)-5-methylcyclohexyl |
| 1.91 | $C_3H_7$-iso | 2-(1-Methylethyl)-5-methylcyclohexyl |
| 1.92 | $C_4H_9$-sek | 2-(1-Methylethyl)-5-methylcyclohexyl |
| 1.93 | $CH_3$ | 2,4,5-Trichlorphenyl |
| 1.94 | $C_2H_5$ | 2,4,5-Trichlorphenyl |

Tabelle 1 (Fortsetzung)

| Verb. Nr. | $R_2$ | $R_3$ |
|---|---|---|
| 1.95 | $C_3H_7$-iso | 2,4,5-Trichlorphenyl |
| 1.96 | $C_4H_9$-sek | 2,4,5-Trichlorphenyl |
| 1.97 | $CH_3$ | $CH_2CCl_2CF_3$ |
| 1.98 | $C_2H_5$ | $CH_2CCl_2CF_3$ |
| 1.99 | $C_3H_7$-iso | $CH_2CCl_2CF_3$ |
| 1.100 | $C_4H_9$-sek | $CH_2CCl_2CF_3$ |
| 1.101 | $CH_3$ | Tetrahydrofuran-2-ylmethyl |
| 1.102 | $C_2H_5$ | Tetrahydrofuran-2-ylmethyl |
| 1.103 | $C_3H_7$-iso | Tetrahydrofuran-2-ylmethyl |
| 1.104 | $C_4H_9$-sek | Tetrahydrofuran-2-ylmethyl |
| 1.105 | $CH_3$ | Tetrahydropyran-2-ylmethyl |
| 1.106 | $C_2H_5$ | Tetrahydropyran-2-ylmethyl |
| 1.107 | $C_3H_7$-iso | Tetrahydropyran-2-ylmethyl |
| 1.108 | $C_4H_9$-sek | Tetrahydropyran-2-ylmethyl |
| 1.109 | $CH_3$ | 1,3-Dioxolan-4-ylmethyl |
| 1.110 | $C_2H_5$ | 1,3-Dioxolan-4-ylmethyl |
| 1.111 | $C_3H_7$-iso | 1,3-Dioxolan-4-ylmethyl |
| 1.112 | $C_4H_9$-sek | 1,3-Dioxolan-4-ylmethyl |
| 1.113 | $CH_3$ | Fluorenylmethyl |
| 1.114 | $C_2H_5$ | Fluorenylmethyl |
| 1.115 | $C_3H_7$-iso | Fluorenylmethyl |
| 1.116 | $C_4H_9$-sek | Fluorenylmethyl |

Tabelle 2

Typische Vertreter von Verbindungen der Formel I, worin $R_1$ tert.Butyldimethylsilyl ist.

| Verb. Nr. | $R_2$ | $R_3$ |
|---|---|---|
| 2.1 | $CH_3$ | $CH_3$ |
| 2.2 | $C_2H_5$ | $CH_3$ |
| 2.3 | $C_3H_7$-iso | $CH_3$ |
| 2.4 | $C_4H_9$-sek | $CH_3$ |
| 2.5 | $CH_3$ | $C_2H_5$ |
| 2.6 | $C_2H_5$ | $C_2H_5$ |
| 2.7 | $C_3H_7$-iso | $C_2H_5$ |
| 2.8 | $C_4H_9$-sek | $C_2H_5$ |
| 2.9 | $CH_3$ | $CH(CH_3)_2$ |
| 2.10 | $C_2H_5$ | $CH(CH_3)_2$ |
| 2.11 | $C_3H_7$-iso | $CH(CH_3)_2$ |
| 2.12 | $C_4H_9$-sek | $CH(CH_3)_2$ |
| 2.13 | $CH_3$ | $C_4H_9$-tert. |
| 2.14 | $C_2H_5$ | $C_4H_9$-tert. |
| 2.15 | $C_3H_7$-iso | $C_4H_9$-tert. |
| 2.16 | $C_4H_9$-sek | $C_4H_9$-tert. |
| 2.17 | $CH_3$ | $CH_2CH_2Br$ |
| 2.18 | $C_2H_5$ | $CH_2CH_2Br$ |
| 2.19 | $C_3H_7$-iso | $CH_2CH_2Br$ |
| 2.20 | $C_4H_9$-sek | $CH_2CH_2Br$ |
| 2.21 | $CH_3$ | $CH_2CH_2Cl$ |
| 2.22 | $C_2H_5$ | $CH_2CH_2Cl$ |
| 2.23 | $C_3H_7$-iso | $CH_2CH_2Cl$ |
| 2.24 | $C_4H_9$-sek | $CH_2CH_2Cl$ |
| 2.25 | $CH_3$ | $CH_2CCl_3$ |
| 2.26 | $C_2H_5$ | $CH_2CCl_3$ |
| 2.27 | $C_3H_7$-iso | $CH_2CCl_3$ |
| 2.28 | $C_4H_9$-sek | $CH_2CCl_3$ |
| 2.29 | $CH_3$ | $CCl_3$ |
| 2.30 | $C_2H_5$ | $CCl_3$ |
| 2.31 | $C_3H_7$-iso | $CCl_3$ |
| 2.32 | $C_4H_9$-sek | $CCl_3$ |
| 2.33 | $CH_3$ | $C(CH_3)_2CCl_3$ |
| 2.34 | $C_2H_5$ | $C(CH_3)_2CCl_3$ |
| 2.35 | $C_3H_7$-iso | $C(CH_3)_2CCl_3$ |
| 2.36 | $C_4H_9$-sek | $C(CH_3)_2CCl_3$ |
| 2.37 | $CH_3$ | $CH{=}CH_2$ |
| 2.38 | $C_2H_5$ | $CH{=}CH_2$ |
| 2.39 | $C_3H_7$-iso | $CH{=}CH_2$ |
| 2.40 | $C_4H_9$-sek | $CH{=}CH_2$ |
| 2.41 | $CH_3$ | $CH_2-CH{=}CH_2$ |
| 2.42 | $C_2H_5$ | $CH_2-CH{=}CH_2$ |
| 2.43 | $C_3H_7$-iso | $CH_2-CH{=}CH_2$ |
| 2.44 | $C_4H_9$-sek | $CH_2-CH{=}CH_2$ |
| 2.45 | $CH_3$ | Benzyl |
| 2.46 | $C_2H_5$ | Benzyl |

Tabelle 2: (Fortsetzung)

| Verb. Nr. | $R_2$ | $R_3$ |
|---|---|---|
| 2.47 | $C_3H_7$-iso | Benzyl |
| 2.48 | $C_4H_9$-sek | Benzyl |
| 2.49 | $CH_3$ | p-Nitrobenzyl |
| 2.50 | $C_2H_5$ | p-Nitrobenzyl |
| 2.51 | $C_3H_7$-iso | p-Nitrobenzyl |
| 2.52 | $C_4H_9$-sek | p-Nitrobenzyl |
| 2.53 | $CH_3$ | Phenyl |
| 2.54 | $C_2H_5$ | Phenyl |
| 2.55 | $C_3H_7$-iso | Phenyl |
| 2.56 | $C_4H_9$-sek | Phenyl |
| 2.57 | $CH_3$ | p-Nitrophenyl |
| 2.58 | $C_2H_5$ | p-Nitrophenyl |
| 2.59 | $C_3H_7$-iso | p-Nitrophenyl |
| 2.60 | $C_4H_9$-sek | p-Nitrophenyl |
| 2.61 | $CH_3$ | p-Methoxycarbonylphenyl |
| 2.62 | $C_2H_5$ | p-Methoxycarbonylphenyl |
| 2.63 | $C_3H_7$-iso | p-Methoxycarbonylphenyl |
| 2.64 | $C_4H_9$-sek | p-Methoxycarbonylphenyl |
| 2.65 | $CH_3$ | 2,2-Dimethyl-1,3-dioxolan-4-ylmethyl |
| 2.66 | $C_2H_5$ | 2,2-Dimethyl-1,3-dioxolan-4-ylmethyl |
| 2.67 | $C_3H_7$-iso | 2,2-Dimethyl-1,3-dioxolan-4-ylmethyl |
| 2.68 | $C_4H_9$-sek | 2,2-Dimethyl-1,3-dioxolan-4-ylmethyl |
| 2.69 | $CH_3$ | $CH_2CH(OH)CH_2OH$ |
| 2.70 | $C_2H_5$ | $CH_2CH(OH)CH_2OH$ |
| 2.71 | $C_3H_7$-iso | $CH_2CH(OH)CH_2OH$ |
| 2.72 | $C_4H_9$-sek | $CH_2CH(OH)CH_2OH$ |
| 2.73 | $CH_3$ | $CH_2CH_2OCH_3$ |
| 2.74 | $C_2H_5$ | $CH_2CH_2OCH_3$ |
| 2.75 | $C_3H_7$-iso | $CH_2CH_2OCH_3$ |
| 2.76 | $C_4H_9$-sek | $CH_2CH_2OCH_3$ |
| 2.77 | $CH_3$ | $CH_2CH(C_2H_5)CH_2CH_2CH_2CH_3$ |
| 2.78 | $C_2H_5$ | $CH_2CH(C_2H_5)CH_2CH_2CH_2CH_3$ |
| 2.79 | $C_3H_7$-iso | $CH_2CH(C_2H_5)CH_2CH_2CH_2CH_3$ |
| 2.80 | $C_4H_9$-sek | $CH_2CH(C_2H_5)CH_2CH_2CH_2CH_3$ |
| 2.81 | $CH_3$ | Cyclohexyl |
| 2.82 | $C_2H_5$ | Cyclohexyl |
| 2.83 | $C_3H_7$-iso | Cyclohexyl |
| 2.84 | $C_4H_9$-sek | Cyclohexyl |
| 2.85 | $CH_3$ | $CH_2CH_2CH_2CH_2CH_2CH_3$ |
| 2.86 | $C_2H_5$ | $CH_2CH_2CH_2CH_2CH_2CH_3$ |
| 2.87 | $C_3H_7$-iso | $CH_2CH_2CH_2CH_2CH_2CH_3$ |
| 2.88 | $C_4H_9$-sek | $CH_2CH_2CH_2CH_2CH_2CH_3$ |
| 2.89 | $CH_3$ | 2-(1-Methylethyl)-5-methylcyclohexyl |
| 2.90 | $C_2H_5$ | 2-(1-Methylethyl)-5-methylcyclohexyl |
| 2.91 | $C_3H_7$-iso | 2-(1-Methylethyl)-5-methylcyclohexyl |
| 2.92 | $C_4H_9$-sek | 2-(1-Methylethyl)-5-methylcyclohexyl |
| 2.93 | $CH_3$ | 2,4,5-Trichlorphenyl |
| 2.94 | $C_2H_5$ | 2,4,5-Trichlorphenyl |

Tabelle 2: (Fortsetzung)

| Verb. Nr. | $R_2$ | $R_3$ |
|---|---|---|
| 2.95 | $C_3H_7$-iso | 2,4,5-Trichlorphenyl |
| 2.96 | $C_4H_9$-sek | 2,4,5-Trichlorphenyl |
| 2.97 | $CH_3$ | $CH_2CCl_2CF_3$ |
| 2.98 | $C_2H_5$ | $CH_2CCl_2CF_3$ |
| 2.99 | $C_3H_7$-iso | $CH_2CCl_2CF_3$ |
| 2.100 | $C_4H_9$-sek | $CH_2CCl_2CF_3$ |
| 2.101 | $CH_3$ | Tetrahydrofuran-2-ylmethyl |
| 2.102 | $C_2H_5$ | Tetrahydrofuran-2-ylmethyl |
| 2.103 | $C_3H_7$-iso | Tetrahydrofuran-2-ylmethyl |
| 2.104 | $C_4H_9$-sek | Tetrahydrofuran-2-ylmethyl |
| 2.105 | $CH_3$ | Tetrahydropyran-2-ylmethyl |
| 2.106 | $C_2H_5$ | Tetrahydropyran-2-ylmethyl |
| 2.107 | $C_3H_7$-iso | Tetrahydropyran-2-ylmethyl |
| 2.108 | $C_4H_9$-sek | Tetrahydropyran-2-ylmethyl |
| 2.109 | $CH_3$ | 1,3-Dioxolan-4-ylmethyl |
| 2.110 | $C_2H_5$ | 1,3-Dioxolan-4-ylmethyl |
| 2.111 | $C_3H_7$-iso | 1,3-Dioxolan-4-ylmethyl |
| 2.112 | $C_4H_9$-sek | 1,3-Dioxolan-4-ylmethyl |
| 2.113 | $CH_3$ | Fluorenylmethyl |
| 2.114 | $C_2H_5$ | Fluorenylmethyl |
| 2.115 | $C_3H_7$-iso | Fluorenylmethyl |
| 2.116 | $C_4H_9$-sek | Fluorenylmethyl |

Der Inhalt der Tabellen 1 und 2 besitzt illustrativen Charakter und stellt keine Begrenzung dar.

Formulierungsbeispiele für den Wirkstoff der Formel I

(% = Gewichtsprozent)

| Spritzpulver | a) | b) | c) |
|---|---|---|---|
| Wirkstoff aus Tabelle 1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykol-ether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| Emulsions-Konzentrat | |
|---|---|
| Wirkstoff aus Tabelle 1 | 10 % |
| Octylphenolpolyethylenglykolether (4-5 Mol AeO) | 3 % |
| Ca-Dodecylbenzolsulfonat | 3 % |
| Ricinusölpolyglykolether (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Stäubemittel | a) | b) |
|---|---|---|
| Wirkstoff aus Tabelle 1 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

Extruder Granulat

| | |
|---|---|
| Wirkstoff aus Tabelle 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

Tabletten, Pellets

| | | |
|---|---|---|
| I | Wirkstoff aus Tabelle 1 | 33,0 % |
| | Methylcellulose | 0,80 % |
| | Kieselsäure hochdispers | 0,80 % |
| | Maisstärke | 8,40 % |

Methylcellulose in Wasser einrühren und quellen lassen; Kieselsäure in die Quellung einrühren und homogen suspendieren. Wirkstoff und Maisstärke mischen. In diese Mischung die wässrige Suspension einarbeiten und zu einem Teig kneten. Diese Masse durch ein Sieb (Maschenweite 12 M) granulieren und dann trocknen.

| | | |
|---|---|---|
| II | Milchzucker krist. | 22,50 % |
| | Maisstärke | 17,00 % |
| | mikrokrist. Cellulose | 16,50 % |
| | Magnesiumstearat | 1,00 % |

Alle 4 Hilfsstoffe gut mischen
Phasen I und II mischen und zu Tabletten oder Pellets verpressen.

Die vorstehend beschriebenen Formulierungen können beispielsweise auch mit einem Wirkstoff aus Tabelle 2 hergestellt werden.

Bei einer Verwendung von Verbindungen der Formel I oder entsprechender Mittel zur Bekämpfung von endoparasitären Nematoden, Cestoden und Trematoden bei Haus- und Nutztieren, wie Rindern, Schafen, Ziegen, Katzen und Hunden, können die Verbindungen oder Mittel den Tieren sowohl als Einzeldosis wie auch wiederholt verabreicht werden, wobei die einzelnen Gaben je nach Tierart vorzugsweise zwischen 0,1 und 10 mg pro kg Körpergewicht betragen. Durch eine protrahierte Verabreichung erzielt man in manchen Fällen eine bessere Wirkung oder man kann mit geringeren Gesamtdosen auskommen. Der Wirkstoff bzw. die ihn enthaltenden Mittel können auch dem Futter oder den Tränken zugesetzt werden. Das Fertigfutter enthält die Wirkstoffkombinationen vorzugsweise in einer Konzentration von 0,005 bis 0,1 Gew. %. Die Mittel können in Form von Lösungen, Emulsionen, Suspensionen, Pulver, Tabletten, Pellets, Bolussen oder Kapseln peroral den Tieren verabreicht werden. Soweit die physikalischen und toxikologischen Eigenschaften von Lösungen oder Emulsionen dies zulassen, können die Verbindungen der Formel I bzw. die sie enthaltenden Mittel den Tieren auch beispielsweise subcutan injiziert, intraruminal verabreicht oder mittels der Pour-on-Methode auf den Körper der Tiere appliziert werden. Ferner ist eine Verabreichung des Wirkstoffs an die Tiere auch durch Lecksteine (Salz) oder Molasse-Blöcke möglich.

## Biologische Beispiele

### Beispiel B.1: Milbizide Wirkung gegen Dermanyssus gallinae

2 bis 3 ml einer Testlösung (250, 100 und 10 ppm Aktivsubstanz) werden in einen nach oben offenen Glasbehälter gegeben und ca. 200 Milben in unterschiedlichen Entwicklungsstadien in diesen Behälter eingesetzt. Der Glasbehälter wird mit einem Wattebausch verschlossen und 10 Minuten gleichmässig, bis zur vollständigen [benetzung der Milben geschüttelt. Danach wird der Behälter umgekehrt hingestellt, bis die überschüssige Testlösung von der Watte aufgesogen ist. Der Behälter wird erneut gekehrt und die behandelten

Zecken zur Evaluierung der Wirksamkeit der Testsubstanzen drei Tage unter Laborbedingungen beobachtet, wobei die Mortalität als Massstab für Wirksamkeit herangezogen wird.

Verbindungen aus den Herstellungsbeispielen zeigen bei 100 ppm eine Wirkung von 100 %. Diese Wirkung wird bei manchen Verbindungen, wie beispielsweise den Verbindungen Nr. 1.2, 1.3, 1.10, 1.18, 1.39, 1.43 und 1.46, bereits mit geringeren Wirkstoffkonzentrationen erzielt.

Beispiel B.2: Wirkung gegen $L_1$-Larven von Lucilia sericata
1 ml einer wässrigen Suspension der zu prüfenden Aktivsubstanz werden so mit 3 ml eines speziellen Larvenzuchtmediums bei ca. 50°C vermischt, dass ein Homogenisat von wahlweise 250 ppm oder 100 ppm Wirkstoffgehalt entsteht. In jede Reagenzglas-Probe werden ca. 30 Lucilia-Larven ($L_1$) eingesetzt. Nach 4 Tagen wird die Mortalitätsrate bestimmt. Die Verbindungen aus den Herstellungsbeispielen zeigen bei 250 ppm eine Wirkung von 100 %, wobei Verbindungen aus den Herstellungsbeispielen diese Wirkung auch noch bei der reduzierten Wirkstoffkonzentration von 100 ppm erzielen, wie beispielsweise die Verbindungen Nr. 1.10, 1.18, 1.26, 1.34, 1.39, 1.43, 1.46, 1.54, 1.58, 1.62, 1.82, 1.90 und 1.114.

Beispiel B.3: Wirkung gegen $L_1$-Larven von Lucilia cuprina
Der Test wird wie unter B.2 beschrieben durchgeführt, jedoch mit Larven von Lucilia cuprina. Die Verbindungen aus den Herstellungsbeispielen bewirken bei 100 ppm eine vollständige Vernichtung des Schädlings. Bei manchen Verbindungen wird dieser Effekt bereits mit niedrigeren Konzentrationen erreicht, beispielsweise mit den Verbindungen Nr. 1.34, 1.90 und 1.114.

Beispiel B.4: Bodennematizide Wirkung gegen Meloidogyne incognita
Eier von Meloidogyne incognita werden in Sand eingemischt. Dieses Gemisch wird dann in 200 ml fassende Tontöpfe eingebracht (5000 Eier pro Topf). Am gleichen Tag wird pro Topf eine 3 Wochen alte Tomatenpflanze gepflanzt und der aus 50 %-igem Emulsionskonzentrat formulierte Wirkstoff mittels Druckapplikation in die Töpfe hineingegeben. Die eingetopften Pflanzen werden nun bei einer Temperatur von 26±1°C und einer relativen Luftfeuchtigkeit von 60 % in einem Gewächshaus aufgestellt. Nach Ablauf von 4 Wochen erfolgt eine Evaluierung durch Untersuchung der Pflanzen auf Wurzelgallbildung nach dem sogenannten Wurzelgall-Index ("Knot Index").

Die Verbindungen der Herstellungsbeispiele zeigen gegen Meloidogyne incognita durch weitgehende Reduktion der Wurzelgallbildung gute Wirksamkeit. Unbehandelte jedoch infizierte Kontrollpflanzen weisen dagegen starke Wurzelgallbildung auf (= 100 %). So hemmen z.B. die Verbindungen aus den Herstellungsbeispielen im obigen Versuch die Wurzelgallbildung fast vollständig (bis zu 10 % Restbefall) oder vollständig. Um eine fast vollständige Hemmung der Reproduktion zu erzielen, genügen bei manchen Verbindungen Konzentrationen von 1 ppm oder weniger, wie beispielsweise bei den Verbindungen Nr. 1.3, 1.26, 1.39, 1.43, 1.70, 1.90 und besonders Nr. 1.2 und 1.66.

Beispiel B.5: Bodennematizide Wirkung gegen Caenorhabditis elegans

Der Test wird wie unter B.4 beschrieben durchgeführt, jedoch mit Caenorhabditis elegans. Die Verbindungn der Herstellungsbeispiele zeigen in diesem Test eine gute Wirksamkeit. Um eine fast vollständige Hemmung der Reproduktion zu erreichen, genügen bei manchen Verbindungen Konzentrationen von 1 ppm und weniger, wie beispielsweise bei den Verbindungen Nr. 1.39, 1.70 und 1.90 und vor allem Nr. 1.2, 1.3, 1.26, 1.43 und 1.66.

Beispiel B.6: Insektizide Frassgift-Wirkung bei Spodoptera littoralis

Eingetopfte Baumwollpflanzen im 5-Blatt-Stadium werden mit einer acetonisch/wässrigen Versuchslösung besprüht, die 3, 12,5 oder 50 ppm der zu prüfenden Verbindung enthält.

Nach dem Antrocknen des Belags werden die Pflanzen mit ca. 30 Larven ($L_1$-Stadium) von Spodoptera littoralis besetzt. Pro Versuchsverbindung und pro Test-Spezies verwendet man zwei Pflanzen. Der Versuch

wird bei ca. 24°C und 60 % relativer Luftfeuchtigkeit durchgeführt. Auswertungen und Zwischenauswertungen auf moribunde Tiere, Wachstum und Larven und Frassschäden erfolgen nach 24 Stunden, 48 Stunden und 72 Stunden.

Verbindungen der Formeln I aus den Herstellungsbeispielen erzielten bereits bei einer Wirkstoffkonzentration von 12,5 ppm eine vollständige Abtötung nach 24 Stunden. Bei manchen Verbindungen wird dieser Effekt bereits mit niedrigeren Konzentrationen erreicht, beispielsweise mit den Verbindungen Nr. 1.3, 1.10, 1.26, 1.34, 1.54, 1.82 und 1.98.

Beispiel B.7: Wirkung gegen pflanzenschädigende Akariden OP-sensible Tetranychus urticae

Die Primärblätter von Bohnenpflanzen (Phaseolus vulgaris) werden 16 Stunden vor dem Versuch mit einem durch T. urticae infestierten, aus einer Massenzucht stammenden Blattstück belegt. Die so mit allen Milben-Stadien infestierten Pflanzen werden nach Entfernung des Blattstücks mit einer Versuchslösung bis zur Tropfnässe besprüht, die wahlweise 3,0 ppm oder 1,6 ppm der zu prüfenden Verbindung enthält. Die Temperatur in der Gewächshauskabine beträgt ca. 25°C.

Nach sieben Tagen wird unter dem Binokular auf Prozentsatz mobiler Stadien (Adulte und Nymphen) und auf vorhandene Eier ausgewertet.

Verbindungen der Formel I erzielten bei einer Wirkstoffkonzentration von 1,6 ppm vollständige Abtötung. Bei manchen Verbindungen wird dieser Effekt bereits mit niedrigeren Konzentrationen erreicht, beispielsweise mit den Verbindungen Nr. 1.3, 1.10, 1.34, 1.82 und 1.98.

Beispiel B.8: Versuch an mit Nematoden (Haemonchus concortus und Trichostrongylus colubriformis) infizierten Schafen

Der Wirkstoff wird als Suspension formuliert mit einer Magensonde oder durch Injektion in den Pansen eines Schafes gegeben, das mit Haemonchus concortus und Trychostrongylus colubriformis künstlich infiziert worden ist. Pro Dosis werden 1 bis 3 Tiere verwendet. Jedes Schaf wird nur einmal mit einer einzigen Dosis behandelt, und zwar wahlweise mit 1 mg oder 0,5 mg/kg Körpergewicht. Die Evaluierung erfolgt durch Vergleich der Anzahl der vor und nach Behandlung im Kot der Schafe ausgeschiedenen Wurmeier.

Gleichzeitig und gleichartig infizierte aber unbehandelte Schafe dienen als Kontrolle. Schafe, die mit einer der Verbindungen der Formeln I bei 1 mg/kg behandelt wurden, zeigten im Vergleich zu unbehandelten, aber infizierten Vergleichsgruppen keinen Nematodenbefall (= komplette Reduktion der Wurmeier im Kot). Verbindungen aus den Herstellungsbeispielen bewirkten dies auch noch bei 0,5 mg/kg.

Beispiel B.9: Kontaktwirkung auf Aphis craccivora

Erbsenkeimlinge, die mit sämtlichen Entwicklungsstadien der Laus infiziert sind, werden mit einer aus einem Emulsionskonzentrat hergestellten Wirkstofflösung besprüht, die wahlweise 50 ppm, 25 ppm oder 12,5 ppm Wirkstoff enthält. Nach 3 Tagen wird auf mehr als 80 % tote bzw. abgefallene Blattläuse ausgewertet. Nur bei dieser Wirkungshöhe wird ein Präparat als wirksam eingestuft.

Verbindungen aus den Herstellungsbeispielen erzielten bei einer Konzentration von 25 ppm eine vollständige Abtötung (= 100 %). Bei manchen Verbindungen wird dieser Effekt bereits mit niedrigeren Konzentrationen erreicht, beispielsweise mit den Verbindungen Nr. 1.2, 1.3, 1.10, 1.34, 1.39, 1.43 und 1.54.

Beispiel B.10: Larvizidwirkung gegen Aedes aegypti

Auf die Oberfläche von 150 ml Wasser, das sich in einem Behälter befindet, wird soviel einer 0,1 %igen acetonischen Lösung des Wirkstoffes pipettiert, dass Konzentrationen von wahlweise 12,5 ppm, 3,3 ppm und 1,6 ppm erhalten werden. Nach Verdunsten des Acetons wird der Behälter mit ca. 30-40 3 Tage alten Aedes-Larven beschickt. Nach 1, 2 und 5 Tagen wird die Mortalität geprüft.

Verbindungen aus den Herstellungsbeispielen bewirkten in diesem Test bei einer Konzentration von 3 ppm bereits nach einem Tag eine vollständige Abtötung sämtlicher Larven. Bei manchen Verbindungen wird dieser Effekt bereits mit niedrigeren Konzentrationen erreicht, beispielsweise mit den Verbindungen Nr. 1.10, 1.26, 1.34, 1.54, 1.58, 1.62, 1.82, 1.90, 1.98 und 1.114.

Patentansprüche

1. Verbindungen der Formel I

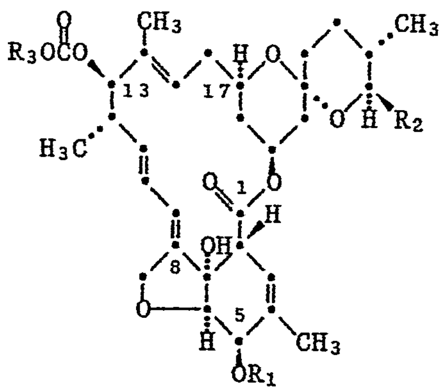

(I)

in welcher

$R_1$ Wasserstoff, eine Silyl- oder Acylgruppe oder $R_4$,

$R_2$ Methyl, Ethyl, Isopropyl oder sek.Butyl und

$R_3$ unsubstituierte oder substituierte gerad- oder verzweigtkettige $C_1$-$C_{18}$-Alkyl-Gruppen, unsubstituierte oder substituierte cycloaliphatische Gruppen mit 3 bis 10 Kohlenstoffatomen, unsubstituierte oder substituierte $C_2$-$C_6$-Alkenyl-Gruppen, unsubstituierte oder substituierte $C_2$-$C_6$-Alkinyl-Gruppen, unsubstituiertes oder substituiertes Phenyl oder unsubstituiertes oder substituiertes Benzyl bedeuten.

2. Verbindungen der Formel I nach Anspruch 1, worin $R_1$ für Wasserstoff oder tert.-Butyldimethylsilyl, $R_2$ für Methyl, Ethyl, Isopropyl oder sek.-Butyl und $R_3$ für $C_1$-$C_{10}$-Alkyl, welches unsubstituiert oder durch 1 bis 6 Halogenatome substituiert oder durch Hydroxyl oder $C_1$-$C_4$-Alkoxy mono- bis trisubstituiert oder durch ein monocyclisches, 5- oder 6-gliedriges, 1 oder 2 Sauerstoffatome enthaltendes, unsubstituiertes oder durch $C_1$-$C_3$-Alkyl ein- bis vierfach substituiertes Oxacycloalkyl monosubstituiert ist; für $C_3$-$C_6$-Cycloalkyl, welches unsubstituiert oder durch $C_1$-$C_3$-Alkyl mono- bis trisubstituiert ist; für $C_2$-$C_4$-Alkenyl; für Phenyl, welches unsubstituiert oder durch Halogen mono- bis trisubstituiert oder durch

$C_1$-$C_3$-Alkoxycarbonyl oder Nitro mono- oder disubstituiert ist; oder für Benzyl, welches unsubstituiert oder durch Nitro mono- oder disubstituiert ist, steht.

3. Verbindungen der Formel I nach Anspruch 2, worin $R_1$ für Wasserstoff oder tert.-Butyldimethylsilyl, $R_2$ für Methyl, Ethyl, Isopropyl oder sek.-Butyl und $R_3$ für $C_1$-$C_8$-Alkyl, welches unsubstituiert oder durch 1 bis 6 Halogenatome, ausgewählt aus der Gruppe bestehend aus Chlor-, Fluor- und Bromatomen, substituiert oder durch Hydroxyl oder Methoxy mono- bis trisubstituiert oder durch ein monocyclisches, 5- oder 6-gliedriges, 1 oder 2 Sauerstoffatome enthaltendes, unsubstituiertes oder durch Methyl ein- bis vierfach substituiertes Oxacycloalkyl monosubstituiert ist; für Cyclohexyl, welches unsubstituiert oder durch $C_1$-$C_3$-Alkyl mono- bis trisubstituiert ist; für $C_2$-$C_4$-Alkenyl; für Phenyl, welches unsubstituiert oder durch Chlor mono- bis trisubstituiert oder durch Methoxycarbonyl oder Nitro mono- oder disubstituiert ist; oder für Benzyl, welches unsubstituiert oder durch Nitro mono- oder disubstituiert ist, steht.

4. Verbindungen der Formel I nach Anspruch 1, worin $R_1$ für Wasserstoff oder tert.-Butyldimethylsilyl, $R_2$ für Methyl, Ethyl, Isopropyl oder sek.-Butyl und $R_3$ für unsubstituiertes $C_1$-$C_8$-Alkyl; für $C_1$-$C_4$-Alkyl, welches durch 1 bis 5 Halogenatome substituiert oder durch Hydroxyl oder $C_1$-$C_3$-Alkoxy mono- oder disubstituiert ist; für Methyl, welches durch ein Oxacycloalkyl, ausgewählt aus der Gruppe bestehend aus Tetrahydrofuranyl, 1,3-Dioxolanyl und Tetrahydropyranyl, monosubstituiert ist, wobei das Oxacycloalkyl unsubstituiert oder durch Methyl ein- bis vierfach substituiert ist; für Cyclohexyl, welches unsubstituiert oder durch $C_1$-$C_3$-Alkyl mono- oder disubstituiert ist; für $C_2$-$C_4$-Alkenyl; für Phenyl, welches unsubstituiert oder durch Chlor mono- bis trisubstituiert oder durch Methoxycarbonyl oder Nitro mono- oder disubstituiert ist; oder für Benzyl, welches unsubstituiert oder durch Nitro mono- oder disubstituiert ist, steht.

5. Verbindungen der Formel I nach Anspruch 4, worin $R_1$ für Wasserstoff, $R_2$ für Methyl, Ethyl oder Isopropyl und $R_3$ für unsubstituiertes $C_1$-$C_8$-Alkyl; für $C_1$-$C_4$-Alkyl, welches durch 1 bis 5 Halogenatome, ausgewählt aus der Gruppe bestehend aus Chlor-, Fluor- und Bromatomen, substituiert oder durch Hydroxyl oder Methoxy mono- oder disubstituiert ist; für Methyl, welches durch ein Oxacycloalkyl, ausgewählt aus der Gruppe bestehend aus Tetrahydrofuranyl, 1,3-Dioxolanyl und Tetrahydropyranyl, monosubstituiert ist, wobei das Oxacycloalkyl unsubstituiert oder durch Methyl ein- bis vierfach substituiert ist; für Cyclohexyl, welches unsubstituiert oder durch $C_1$-$C_3$-Alkyl mono-oder disubstituiert ist; für $C_2$-$C_4$-Alkenyl; für Phenyl, welches unsubstituiert oder durch Chlor mono- bis trisubstituiert oder durch Methoxycarbonyl oder Nitro mono- oder disubstituiert ist; oder für Benzyl, welches unsubstituiert oder durch Nitro mono- oder disubstituiert ist, steht.

6. Verbindungen der Formel I nach Anspruch 1, worin $R_1$ für Wasserstoff oder tert.-Butyldimethylsilyl, $R_2$ für Methyl, Ethyl oder Isopropyl und $R_3$ für $C_1$-$C_3$-Alkyl, welches unsubstituiert oder durch Chlor mono-bis trisubstituiert oder durch Hydroxy mono- oder disubstituiert oder durch 1,3-Dioxolanyl, welches unsubstituiert oder durch Methyl ein- bis vierfach substituiert ist, monosubstituiert ist; für Cyclohexyl; oder für $C_2$-$C_4$-Alkenyl steht.

7. Verbindungen der Formel I nach Anspruch 6, worin $R_1$ für Wasserstoff, $R_2$ für Methyl, Ethyl oder Isopropyl und $R_3$ für $C_1$-$C_3$-Alkyl, welches unsubstituiert oder durch Chlor mono- bis trisubstituiert oder durch Hydroxy mono- oder disubstituiert oder durch 1,3-Dioxolanyl oder 2,2-Dimethyl-1,3-dioxolanyl monosubstituiert ist; für Cyclohexyl; oder für Vinyl oder Allyl steht.

8. Verbindungen der Formel I nach Anspruch 1, worin $R_1$ für Wasserstoff oder tert.-Butyldimethylsilyl, $R_2$ für Methyl, Ethyl oder Isopropyl und $R_3$ für Methyl, (2,2-Dimethyl-1,3-dioxolan-4-yl)-methyl, Vinyl oder Allyl steht.

9. Verbindungen der Formel I nach Anspruch 8, worin $R_1$ für Wasserstoff, $R_2$ für Isopropyl und $R_3$ für Methyl, Vinyl oder Allyl steht.

10. Verbindungen der Formel I nach Anspruch 1, worin $R_1$ für Wasserstoff, $R_2$ für Methyl oder Ethyl und $R_3$ für Methyl, Ethyl, 2,2,2-Trichlorethyl, 2,3-Dihydroxypropyl, (2,2-Dimethyl-1,3-dioxolan-4-yl)-methyl oder Cyclohexyl steht.

11. Verbindungen der Formel I nach Anspruch 1, worin $R_1$ für tert.-Butyldimethylsilyl, $R_2$ für Methyl oder Ethyl und $R_3$ für Methyl oder Cyclohexyl steht.

12. Verbindungen der Formel I nach Anspruch 1, worin $R_1$ für Wasserstoff, $R_2$ für Methyl, Ethyl oder Isopropyl und $R_3$ für Methyl, Ethyl, 1,1-Dimethyl-2,2,2-trichlorethyl, (2,2-Dimethyl-1,3-dioxolan-4-yl)-methyl oder Cyclohexyl steht.

13. Eine Verbindung der Formel I nach Anspruch 12, ausgewählt aus der Gruppe
13β-(Methoxycarbonyloxy)-milbemycin $A_3$
13β-(Methoxycarbonyloxy)-milbemycin $A_4$
13β-(Methoxycarbonyloxy)-milbemycin D
13β-(Ethoxycarbonyloxy)-milbemycin $A_3$
13β-(Ethoxycarbonyloxy)-milbemycin $A_4$
13β-(Ethoxycarbonyloxy)-milbemycin D
13β-[(1,1-Dimethyl-2,2,2-trichlorethoxy)-carbonyloxy]-milbemycin $A_3$
13β-[(1,1-Dimethyl-2,2,2-trichlorethoxy)-carbonyloxy]-milbemycin $A_4$
13β-[(1,1-Dimethyl-2,2,2-trichlorethoxy)-carbonyloxy]-milbemycin D
13β-[(2,2-Dimethyl-1,3-dioxolan-4-yl)-methoxycarbonyloxy]-milbemycin $A_3$
13β-[(2,2-Dimethyl-1,3-dioxolan-4-yl)-methoxycarbonyloxy]-milbemycin $A_4$
13β-[(2,2-Dimethyl-1,3-dioxolan-4-yl)-methoxycarbonyloxy]-milbemycin D
13β-(Cyclohexyloxycarbonyloxy)-milbemycin $A_3$
13β-(Cyclohexyloxycarbonyloxy)-milbemycin $A_4$ und

13β-(Cyclohexyloxycarbonyloxy)-milbemycin D.

14. Verbindungen der Formel I nach Anspruch 1, worin $R_1$ für tert.-Butyldimethylsilyl, $R_2$ für Methyl, Ethyl oder Isopropyl und $R_3$ für Methyl, Ethyl, 1,1-Dimethyl-2,2,2-trichlorethyl, (2,2-Dimethyl-1,3-dioxolan-4-yl)-methyl oder Cyclohexyl steht.

15. Eine Verbindung der Formel I nach Anspruch 14, ausgwählt aus der Gruppe

5-O-tert.-Butyldimethylsilyl-13β-(methoxycarbonyloxy)-milbemycin $A_3$
5-O-tert.-Butyldimethylsilyl-13β-(methoxycarbonyloxy)-milbemycin $A_4$
5-O-tert.-Butyldimethylsilyl-13β-(methoxycarbonyloxy)-milbemycin D
5-O-tert.-Butyldimethylsilyl-13β-(ethoxycarbonyloxy)-milbemycin $A_3$
5-O-tert.-Butyldimethylsilyl-13β-(ethoxycarbonyloxy)-milbemycin $A_4$
5-O-tert.-Butyldimethylsilyl-13β-(ethoxycarbonyloxy)-milbemycin D
5-O-tert.-Butyldimethylsilyl-13β-[(1,1-dimethyl-2,2,2-trichlorethoxy)-carbonyloxy]-milbemycin $A_3$
5-O-tert.-Butyldimethylsilyl-13β-[(1,1-dimethyl-2,2,2-trichlorethoxy)-carbonyloxy]-milbemycin $A_4$
5-O-tert.-Butyldimethylsilyl-13β-[(1,1-dimethyl-2,2,2-trichlorethoxy)-carbonyloxy]-milbemycin D
5-O-tert.-Butyldimethylsilyl-13β-[(2,2-dimethyl-1,3-dioxolan-4-yl)-methoxycarbonyloxy]-milbemycin $A_3$
5-O-tert.-Butyldimethylsilyl-13β-[(2,2-dimethyl-1,3-dioxolan-4-yl)-methoxycarbonyloxy]-milbemycin $A_4$
5-O-tert.-Butyldimethylsilyl-13β-[(2,2-dimethyl-1,3-dioxolan-4-yl)-methoxycarbonyloxy]-milbemycin D
5-O-tert.-Butyldimethylsilyl-13β-(cyclohexyloxycarbonyloxy)-milbemycin $A_3$
5-O-tert.-Butyldimethylsilyl-13β-(cyclohexyloxycarbonyloxy)-milbemycin $A_4$ und
5-O-tert.-Butyldimethylsilyl-13β-(cyclohexyloxycarbonyloxy)-milbemycin D.

16. Verbindungen der Formel I nach Anspruch 1, worin $R_1$ für Acetyl, $R_2$ für Methyl, Ethyl oder Isopropyl und $R_3$ für Methyl, Ethyl, 1,1-Dimethyl-2,2,2-trichlorethyl, (2,2-Dimethyl-1,3-dioxolan-4-yl)-methyl oder Cyclohexyl steht.

17. Eine Verbindung der Formel I nach Anspruch 16, ausgewählt aus der Gruppe
5-O-Acetyl-13β-(methoxycarbonyloxy)-milbemycin $A_3$
5-O-Acetyl-13β-(methoxycarbonyloxy)-milbemycin $A_4$
5-O-Acetyl-13β-(methoxycarbonyloxy)-milbemycin D
5-O-Acetyl-13β-(ethoxycarbonyloxy)-milbemycin $A_3$
5-O-Acetyl-13β-(ethoxycarbonyloxy)-milbemycin $A_4$
5-O-Acetyl-13β-(ethoxycarbonyloxy)-milbemycin D
5-O-Acetyl-13β-[(1,1-dimethyl-2,2,2-trichlorethoxy)-carbonyloxy]-milbemycin $A_3$
5-O-Acetyl-13β-[(1,1-dimethyl-2,2,2-trichlorethoxy)-carbonyloxy]-milbemycin $A_4$
5-O-Acetyl-13β-[(1,1-dimethyl-2,2,2-trichlorethoxy)-carbonyloxy]-milbemycin D
5-O-Acetyl-13β-[(2,2-dimethyl-1,3-dioxolan-4-yl)-methoxycarbonyl-oxy]-milbemycin $A_3$
5-O-Acetyl-13β-[(2,2-dimethyl-1,3-dioxolan-4-yl)-methoxycarbonyl-oxy]-milbemycin $A_4$
5-O-Acetyl-13β-[(2,2-dimethyl-1,3-dioxolan-4-yl)-methoxycarbonyl-oxy]-milbemycin D
5-O-Acetyl-13β-(cyclohexyloxycarbonyloxy)-milbemycin $A_3$
5-O-Acetyl-13β-(cyclohexyloxycarbonyloxy)-milbemycin $A_4$ und
5-O-Acetyl-13β-(cyclohexyloxycarbonyloxy)-milbemycin D.

18. Verbindungen der Formel I nach Anspruch 1, worin $R_1$ für Wasserstoff oder tert.-Butyldimethylsilyl, $R_2$ für Methyl, Ethyl oder Isopropyl und $R_3$ für Methyl, Vinyl, Allyl oder Cyclohexyl oder zusätzlich, wenn $R_1$ Wasserstoff bedeutet, für Ethyl, 2,2,2-Trichlorethyl, (2,2-Dimethyl-1,3-dioxolan-4-yl)-methyl oder 2,3-Dihydroxy-n-propyl steht.

19. Eine Verbindung der Formel I nach Anspruch 18, ausgewählt aus der Gruppe

13β-(Methoxycarbonyloxy)-milbemycin $A_4$

13β-(Methoxycarbonyloxy)-milbemycin D

13β-(Ethoxycarbonyloxy)-milbemycin $A_4$

13β-[(2,2,2-Trichlorethoxy)-carbonyloxy]-milbemycin $A_4$

13β-(Vinyloxycarbonyloxy)-milbemycin D

13β-(Allyloxycarbonyloxy)-milbemycin D

13β-[(2,2-Dimethyl-1,3-dioxolan-4-yl)-methoxycarbonyloxy]-milbemycin $A_4$

13β-[(2,3-Dihydroxypropoxy)-carbonyloxy]-milbemycin $A_4$

13β-(Cyclohexyloxycarbonyloxy)-milbemycin $A_4$

5-O-tert.-Butyldimethylsilyl-13β-(methoxycarbonyloxy)-milbemycin $A_4$

5-O-tert.-Butyldimethylsilyl-13β-(methoxycarbonyloxy)-milbemycin D

5-O-tert.-Butyldimethylsilyl-13β-(vinyloxycarbonyloxy)-milbemycin D

5-O-tert.-Butyldimethylsilyl-13β-(allyloxycarbonyloxy)-milbemycin D und

5-O-tert.-Butyldimethylsilyl-13β-(cyclohexyloxycarbonyloxy)-milbemycin $A_4$.

20. 13-β-(Tetrahydrofuran-2-ylmethoxycarbonyloxy)-milbemycin $A_4$ als Verbindung der Formel I nach Anspruch 1.

21. Verfahren zur Herstellung von Verbindungen der Formel I

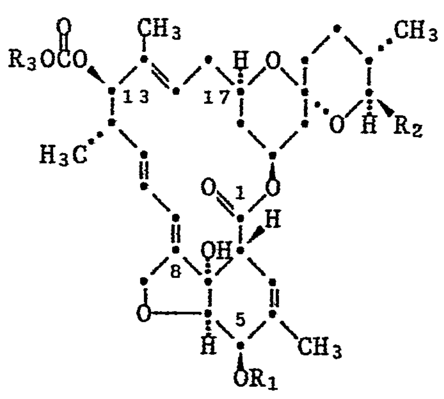

(I)

in welcher

$R_1$ Wasserstoff, eine Silyl- oder Acylgruppe oder $R_4$,

$R_2$ Methyl, Ethyl, Isopropyl oder sek.Butyl und

$R_3$ unsubstituierte oder substituierte gerad- oder

verzweigtkettige $C_1$-$C_{18}$-Alkyl-Gruppen, unsubstituierte oder substituierte cycloaliphatische Gruppen mit 3 bis 10 Kohlenstoffatomen, unsubstituierte oder substituierte $C_2$-$C_6$-Alkenyl-Gruppen, unsubstituierte oder substituierte $C_2$-$C_6$-Alkinyl-Gruppen, unsubstituiertes oder substituiertes Phenyl oder unsubstituiertes oder substituiertes Benzyl bedeuten, dadurch gekennzeichnet, dass man

a) eine Verbindung der Formel II

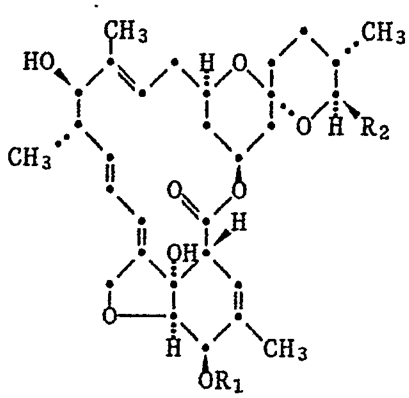

(II),

worin $R_1$ und $R_2$ die unter Formel I angegebenen Bedeutungen besitzen, mit einer zur Einführung einer 13β-Carbonyldioxygruppe $R_3O$-C(O)O- geeigneten Verbindung umsetzt und danach, sofern ein Produkt mit einer freien 5-Hydroxy-Gruppe erwünscht ist, den Substituenten $R_1$ hydrolytisch abspaltet oder

b) eine Verbindung der Formel IV

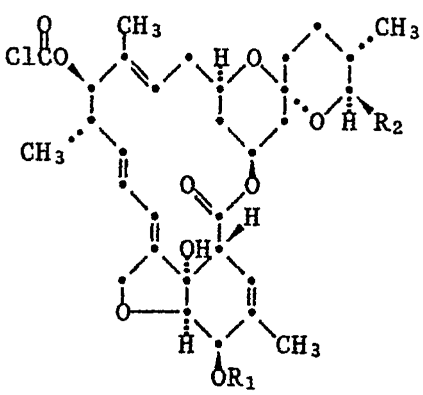

(IV),

worin $R_1$ und $R_2$ die unter Formel I angegebenen Bedeutungen haben, mit einer Verbindung der Formel V,

$$R_3OH \qquad (V),$$

worin $R_3$ wie unter Formel I definiert ist, umsetzt und, sofern ein Produkt mit einer freien 5-Hydroxy-Gruppe erwünscht ist, durch anschliessende hydrolytische Abspaltung den Substituenten $R_1$ entfernt.

22. Verfahren nach Anspruch 21, dadurch gekennzeichnet, dass man eine Verbindung der Formel II, worin $R_1$ und $R_2$ die angegebenen Bedeutungen haben, mit einem Chlorameisensäureester der Formel III

$$R_3O\text{-}C(O)Cl \qquad (III)$$

worin $R_3$ die für Formel I angegebene Bedeutung hat, umsetzt.

23. Mittel zur Bekämpfung von Schädlingen, dadurch gekennzeichnet, dass es neben üblichen Formulierungshilfsstoffen als Wirkstoff mindestens eine Verbindung der Formel I gemäss Anspruch 1 enthält.

24. Verfahren zur Bekämpfung von Schädlingen, dadurch gekennzeichnet, dass man eine pestizid-wirksame Menge mindestens einer Verbindung der Formel I gemäss Anspruch 1 auf oder in die Wirtstiere, Wirtspflanzen oder sonstigen Lebensräume der Schädlinge appliziert.

25. Verfahren nach Anspruch 24, dadurch gekennzeichnet, dass die zu bekämpfenden Schädlinge Tiere befallende Endo- oder Ektoparasiten sind.

26.Verfahren nach Anspruch 24, dadurch gekennzeichnet, dass die zu bekämpfenden Schädlinge pflanzenschädigende Parasiten sind.

FO 7.5 HL/hpw*

Europäisches Patentamt

# EUROPÄISCHER RECHERCHENBERICHT

0245209

Nummer der Anmeldung

EP 87 81 0282

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 142 969 (SANKYO)<br>* Seiten 56-59 *<br>--- | 1,23 | C 07 D 493/22<br>C 07 F 7/04<br>A 61 K 31/35<br>A 01 N 43/90 //<br>(C 07 D 493/22<br>C 07 D 313:00<br>C 07 D 311:00<br>C 07 D 311:00<br>C 07 D 307:00 ) |
| Y | EP-A-0 008 184 (MERCK & CO.)<br>* Seiten 5,6,28-31 *<br>--- | 1,23 | |
| P,X | EP-A-0 184 308 (SANKYO)<br>* Seite 57 *<br>----- | 1-22 | |
| | | | RECHERCHIERTE SACHGEBIETE (Int. Cl.4) |
| | | | C 07 D 493/00<br>A 61 K 31/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 01-07-1987 | VERHULST W. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82